(19) Europäisches Patentamt
European Patent Office
Office européen des brevets

(11) **EP 2 404 599 A1**

(12) **EUROPEAN PATENT APPLICATION**
published in accordance with Art. 153(4) EPC


(43) Date of publication:
**11.01.2012 Bulletin 2012/02**

(21) Application number: **10748372.9**

(22) Date of filing: **05.03.2010**

(51) Int Cl.:
***A61K 31/35*** *(2006.01)* ***A61P 25/28*** *(2006.01)*
***A61P 25/08*** *(2006.01)*

(86) International application number:
**PCT/ES2010/070126**

(87) International publication number:
**WO 2010/100312 (10.09.2010 Gazette 2010/36)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **06.03.2009 ES 200900718**



(71) Applicant: **Neuron Biopharma, S.A.**
**18100 Armilla - Granada (ES)**

(72) Inventors:
- **BURGOS MUÑOZ, Javier Santos**
  **E-18100 Armilla - Granada (ES)**
- **RAMOS MARTÍN, Maria del Carmen**
  **E-18100 Armilla - Granada (ES)**
- **SIERRA ÁVILA, Saleta**
  **E-18100 Armilla - Granada (ES)**
- **ALFARO SÁNCHEZ, Juan María**
  **E-18100 Armilla - Granada (ES)**
- **VELASCO ALVAREZ, Javier**
  **E-18100 Armilla - Granada (ES)**
- **RUMBERO SÁNCHEZ, Ángel**
  **E-18100 Armilla - Granada (ES)**
- **RAMÍREZ MORENO, Carlos**
  **E-18100 Armilla - Granada (ES)**

(74) Representative: **ABG Patentes, S.L.**
**Avenida de Burgos 16D**
**Edificio Euromor**
**28036 Madrid (ES)**


(54) **NEUROPROTECTIVE COMPOUNDS**


(57) The invention describes the use of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid, of formula (I), where R is $CH_3CH_2CO$-, $(CH_3)_2CHCO$- or $(CH_3)_3CCO$-, its hydroxy acid forms and the pharmaceutically acceptable salts of said hydroxy acids, as neuroprotective compounds potentially useful for the prevention and/or treatment of neurodegenerative diseases, or of diseases associated with an unwanted oxidation or of age-associated pathological processes, or of epilepsy, epileptic seizures or convulsions.


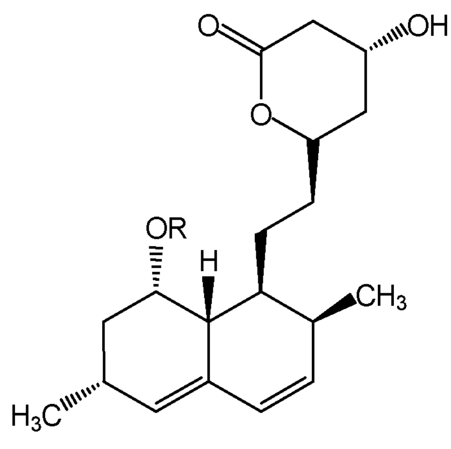

(I)

EP 2 404 599 A1

**Description**

Field of the Invention

The present invention relates to the use of (1*S*,3*R*,7S,8*S*,8a*R*)-1,2,3,7,8,8a-hexahydro-3,7-dimethyl-8-[2-[(2*R*, 4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid, as neuroprotective compounds potentially useful for the prevention and/or treatment of neurodegenerative diseases or of diseases associated with an unwanted oxidation or of age-associated pathological processes, or of epilepsy, convulsive seizures or convulsions.

Background of the Invention

The high incidence of neurodegenerative diseases and of age-associated diseases is a problem of the first order worldwide. It is therefore necessary to search for neuroprotective compounds preventing or palliating said diseases. Of all of them, Alzheimer's disease (AD) is the most prevalent, it being estimated that 81 million people will suffer from this disease in 2040 (Blennow et al., Lancet 2006; 368: 387-403). It is estimated that more than half a million people are currently suffering from AD in Spain alone. The costs associated to this disease are proportionally high, and it is calculated that the total cost derived from caring for Alzheimer's patients is 81,000 and 22,000 million € in the United States and in the United Kingdom, respectively. Currently there are no effective drugs which prevent or impede this disease, therefore it is necessary to search for and validate novel neuroprotective compounds which prevent neuronal damage.

Different strategies are currently being followed for obtaining new compounds, since it has been seen that the current drugs offer little benefits to the patients, temporarily delaying (one year, at best) some symptoms of the illness but not preventing their progress. The current therapeutic options are based on the inhibition of acetylcholinesterase with drugs such as donepezil, galantamine or rivastigmine, or on the capacity of memantine in antagonizing a glutamate receptor, NMDA (N-methyl-D-aspartic acid).

Due to the low success of these drugs, new lines of research have opened up and among them, research on inhibitors of 3-hydroxy-3-methylglutaryl-coenzyme A reductase (HMG-CoA) enzyme (better known as statins) as therapeutic agents stands out in recent years. These compounds are commonly used to reduce the high cholesterol levels associated with low density lipoproteins (LDL). New properties of the statins have recently been described, especially at the level of brain damage caused by trauma or in dementias, new antioxidant and anti-inflammatory activities being proposed (Pahan, Cell Mol Life Sci. 2006; 63[10]:1165-78), and certain statins (e.g., simvastatin) have been demonstrated to intensify the learning and memory capacity in mice (Ling et al., Ann Neurol. 2006; 60[6]:729-39) or protect them against convulsive seizures associated with epileptic phenomena (Lee et al., Neurosci Lett. 2008; 440: 260-4). Furthermore, the statins have also demonstrated their effectiveness in phase II clinical trials which suggest positive results against the treatment of cerebral vasospasm (Fandino et al., Neurocirugia. 2007; 18: 16-27), as well as against neuronal death induced by ischemic damage in the retina (Honjo et al., Arch. Ophthalmol. 2002; 120: 1707-13). Nevertheless, it is currently being discussed whether the neuroprotective effects of the different commercial statins (e.g., atorvastatin, lovastatin, simvastatin, etc.) are due to a direct effect on the lipid metabolism, or whether in contrast they are a result of alternative pathways.

Summary of the Invention

Although there is literature on the potential neuroprotective effect of statins, the authors of this invention have found that non-commercial monacolin J derivatives, specifically (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid, are neuroprotective agents, regardless of their activity on the lipid metabolism.

The neuroprotective activity of said compounds has been clearly shown against different aggressions which cause the production of reactive oxygen species (ROS) and neuronal death due to oxidative stress regardless of their activity on the lipid metabolism (Example 1), and against aggressions which cause neuronal death caused by endoplasmic reticulum stress (Example 2), neuronal death caused by apoptosis (Example 3), death by apoptosis in the brain of the zebrafish embryo (Example 4). It has further been demonstrated that said derivatives cross the blood-brain barrier (Example 5), and that they have a neuroprotective effect in the regions of the brain involved in Alzheimer's disease, such as the hippocampus (Example 6), or the lateral entorhinal nucleus or the amygdaloid nucleus (Example 7). Finally, it has been demonstrated that these compounds act as antiepileptics or anticonvulsants (Example 8).

Said examples clearly show the potential use of said compounds in the prevention and/or treatment of neuronal death associated with neurodegenerative diseases (e.g., Alzheimer's, Parkinson's, multiple sclerosis, lateral amyotrophic

sclerosis, *status epilepticus,* Huntington's, etc.), or of diseases associated with an unwanted oxidation or of age-associated pathological processes.

Therefore, the present invention relates to the use of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid, as neuroprotective agents, particularly neuroprotective against (i) neurodegenerative diseases (e.g., Alzheimer's, Parkinson's, multiple sclerosis, lateral amyotrophic sclerosis, *status epilepticus*, Huntington's, etc.), more specifically, neuroprotective against processes of apoptosis, oxidative stress or endoplasmic reticulum stress associated with said neurodegenerative diseases, or against (ii) diseases associated with an unwanted oxidation, or against (iii) age-associated pathological processes, or against (iv) epilepsy, epileptic seizures or convulsions.

The results obtained in the present invention can be extrapolated for prophylactic or therapeutic purposes for their application on the at-risk population.

Therefore, in one aspect, the invention relates to a (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivative, optionally mono- or di-methylated at the carbon in the alpha position of propanoic acid, for use in the prevention and/or treatment of neurodegenerative diseases, or of diseases associated with an unwanted oxidation, or of age-associated pathological processes, or of epilepsy, epileptic seizures or convulsions.

In another aspect, the invention relates to the use of a (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivative, optionally mono- or di-methylated at the carbon in the alpha position of propanoic acid, in the preparation of a pharmaceutical composition for the prevention and/or treatment of neurodegenerative diseases, or of diseases associated with an unwanted oxidation, or of age-associated pathological processes, or of epilepsy, epileptic seizures or convulsions. In a particular embodiment, said pharmaceutical composition comprises one of said compounds whereas in another particular embodiment, said pharmaceutical composition comprises two or more of said compounds.

In another aspect, the invention relates to a method for the prevention and/or treatment of neurodegenerative diseases, or of diseases associated with an unwanted oxidation, or of age-associated pathological processes, or of epilepsy, epileptic seizures or convulsions, in a subject in need of treatment, comprising the administration to said subject of a therapeutically effective amount of one or more of these compounds.

## Brief Description of the Drawings

Figure 1 is a bar graph depicting the inhibition by (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate (NST0003) of the percentage of reactive oxygen species (ROS) induced by oxidative stress with xanthine/xanthine oxidase (XXO) and normalized by the percentage of intracellular lactate dehydrogenase (LDH) as a measurement of cell viability, in comparison with ascorbic acid (AA) as a specific antioxidant.

Figure 2 is an XY scatter chart depicting the decrease by (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) of the percentage of neuronal death induced by oxidative stress with xanthine/xanthine oxidase (XXO).

Figure 3 is a sigmoid representation depicting the inhibition of the HMG-CoA reductase enzyme by the different (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives (NST0003, NST0004 and NST0005) in comparison with atorvastatin, a specific inhibitor of the HMG-CoA reductase enzyme.

Figure 4 is an XY scatter chart depicting the decrease by (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropionate (NST0005) of the percentage of neuronal death induced by endoplasmic reticulum stress with tunicamycin (Tm).

Figure 5 is an XY scatter chart depicting the decrease by (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate (NST0004) of the percentage of neuronal death induced by apoptosis with camptothecin (Campt).

Figure 6 is a bar graph depicting the inhibition by (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) of the apoptosis induced with camptothecin (Campt) and determined by flow cytometry, in comparison with the specific caspase inhibitor Z-VAD-fmk.

Figure 7 is a bar graph depicting the protection by (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) of the apoptosis induced by camptothecin (Campt) and determined by the measurement of caspase 3/7 activation

in comparison with the specific caspase inhibitor Z-VAD-fmk.

Figure 8 is a bar graph depicting the inhibition by (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate (NST0003), (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate (NST0004) and (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yllethyl-1-naphthalenyl 2,2-dimethylpropanoate (NST0005) of the apoptosis induced with tert-butyl hydroperoxide (TBH) in the brain of the zebrafish embryo.

Figure 9 is an XY scatter chart depicting the effective permeability of the different (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives (NST0003, NST0004 and NST0005) expressed as $P_e$ (in cm/s) versus the crossing of the blood-brain barrier [BBB] (in percentage). The values of these parameters, together with the number of experiments performed (n) and the theoretical lipophilicity index (cLogP), are included in the table.

Figure 10 shows the neuroprotective effect of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) in the hippocampus of mice. The following composition of micrographs is shown: (A) CA1, CA2 and CA3 regions of the hippocampus of mice where the samples have been stained with Fluoro-Jade B (100X magnification); (B) CA1 region of the hippocampus of mice where the microtubule-associated protein 2 (MAP2) is detected (100X); (C) CA3 region of the hippocampus of mice where the samples have been stained with Acridine Orange (200X); (D) CA1, CA2 and CA3 regions of the hippocampus of mice where the samples have been stained with hematoxylin and eosin (100X). The figure represents the histopathological analysis of the neurodegeneration, of the neuritic dystrophy, of the apoptosis and of the necrosis of these regions according to the pretreatment (24 h prior (-24 h) and 0.5 h prior (-0.5 h) to the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 11 shows the neuroprotective effect of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) in the lateral entorhinal nucleus and in the amygdaloid nucleus of the brain of mice. A composition of micrographs showing the detection of the microtubule-associated protein 2 (MAP2) (100X magnification) in the lateral entorhinal nucleus and amygdaloid nucleus of the cerebral cortex of mice is presented. The figure represents the histopathological analysis of the neuritic dystrophy of these regions according to the pretreatment (-24 and -0.5 hours of the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 12 shows the effect of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) against the astrogliosis in the lateral entorhinal nucleus and in the amygdaloid nucleus of the brain of mice. It is presented a composition of micrographs showing the detection of the glial fibrillary acidic protein (GFAP) (100X magnification) in the lateral entorhinal nucleus and amygdaloid nucleus of the cerebral cortex of mice. The figure represents the histopathological analysis of the reactive astrogliosis of these regions according to the pretreatment (-24 and -0.5 hours of the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 13 is a bar graph showing the effect of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) on the latency (or time of onset of the first convulsion) after the inoculation of KA (mean$\pm$SEM of the time in minutes) (y-axis) according to the pretreatment received (x-axis). The group of pretreatment with PBS is represented in black and the group of treatment with NST0005 at 50 mg/kg by weight is represented in gray.

Figure 14 is an XY scatter chart depicting the antiepileptic effect of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) on the level of severity of the epileptogenic symptoms according to Racine's scale (Racine, Electroencephalogr Clin Neurophysiol 1972; 32[3]:281-94), against the post-inoculation time of the epileptogenic substance (kainic acid or kainate or KA). The chart shows the progress of the epileptogenic state of the animals according to the treatment received: PBS is represented with black squares and a black line and the group of treatment with NST0005 at 50 mg/kg by weight is represented with gray circles and a dotted gray line.

## Detailed Description of the Invention

### Definitions

To aid in understanding the invention object of this patent application, the meaning of terms and expressions used in the context of the invention is explained below.

As it is used herein, the term "neuroprotective" relates to the attenuation or disappearance of the effects of neuronal degeneration or death by means of any mechanism known or to be known, for example, necrosis, apoptosis, autophagia, oxidative damage, endoplasmic reticulum damage, deposition of byproducts, loss of cell architecture, etc., or to the reduction or disappearance of the side effects thereof.

As it is used herein, the term "neurodegenerative disease" includes diseases which result from the degeneration or deterioration of nervous tissue, particularly of neurons, leading over time to a dysfunction or to a disability; the term degeneration includes loss of cell viability, loss of cell function and/or loss of the number of cells (neurons or others). Illustrative, non-limiting examples of neurodegenerative diseases include Alzheimer's disease, Huntington's disease, Parkinson's disease, amyotrophic lateral sclerosis (ALS), multiple sclerosis, etc. In a particular embodiment, said neurodegenerative disease is a disease related to neuronal death caused by a substance which, for example, causes oxidative stress or endoplasmic reticulum stress or apoptosis or neuronal death in general.

As it is used herein, the term "disease associated with an unwanted oxidation" relates to a disease caused by unwanted oxidation (e.g., excessive oxidation) or in which said unwanted oxidation is a symptom. Said unwanted oxidation can be a result of the damage caused by free radicals in proteins, DNA and/or lipids independently from the specific free radical involved or from the target. Unwanted oxidation involves an excessive generation of free radicals which can cause a dysfunction in cells, tissues or organs and can therefore form a potential mechanism of a disease. In a particular embodiment, said unwanted oxidation can be caused by age (aging) or by a neurodegenerative process and can cause by itself or in combination with other factors the onset of several diseases. In a specific embodiment, said unwanted oxidation relates to the oxidative damage caused by a substance which causes oxidative stress.

As it is used herein, the term "age-associated pathological process" relates to any age-related event or combination of events causing loss of cell viability of the nervous tissue or cell sensitization of the nervous tissue, loss of cell function and/or loss of the number of cells (neurons or others), including cell metabolic dysfunction, stress processes, infections by pathogens, genetic alterations, genetic susceptibility, trauma, ischemia, epilepsy, etc.

As it is used herein, the term "epilepsy" relates to a chronic brain syndrome having varied causes, characterized by recurrent seizures due to excessive hypersynchronic discharges of nervous impulses by the brain neurons, possibly associated with several clinical and paraclinical manifestations. The seizures can be convulsive or non-convulsive. Epilepsy can have many causes; in some cases it can be due to different types of brain injuries (e.g., brain traumas, sequelae of meningitis, tumors, etc.); in other cases there is no injury but a genetic predisposition to seizures; in other cases, the etiology of the epilepsy can be environmental, due to pharmacological treatments, due to excitotoxicity, trauma, stress processes, aging, development problems, neurological diseases, psychological crises, problems during gestation, problems during labor, etc.

As it is used herein, the term "epileptic or convulsant" relates to any epileptic seizure or convulsion of any etiology, for example, genetic, environmental, due to pharmacological treatments, due to excitotoxicity, due to trauma, due to stress processes, due to aging, due to development problems, due to neurological diseases, due to psychological crises, due to problems during gestation, due to problems during labor, etc. An epileptic seizure occurs when an abnormal electrical activity in the brain causes an involuntary change of body movement or function, in feeling, in the capacity of being alert or in behavior, and can be partial or generalized (convulsive or non-convulsive).

As it is used herein, the term "subject" relates to a member of a mammal species and includes but is not limited to domestic animals, primates and humans; preferably, the subject is a male or female human being of any age or race. In a particular embodiment, said subject is a mammal which suffers, or is susceptible of suffering, age-associated pathological processes, such as aging, or a neurodegenerative disease, such as a chronic neurodegenerative disease.

As it is used herein, the term "pharmaceutically acceptable" relates to the fact that the compound is physiologically tolerable and generally does not cause an allergic reaction or a similar unfavorable reaction, such as a gastric disorder, dizziness or the like, when administered to a subject; said term "pharmaceutically acceptable" preferably means approved by a government regulatory agency or listed in the United States Pharmacopoeia or in another generally recognized pharmacopoeia for use in animals (e.g., European Pharmacopoeia, etc.).

As it is used herein, the term "pharmaceutically acceptable salt" includes "pharmaceutically acceptable metal salts" as well as "pharmaceutically acceptable amine salts". The term "pharmaceutically acceptable metal salt" contemplates salts formed with sodium, potassium, calcium, magnesium, aluminum, iron or zinc ions. The term "pharmaceutically acceptable amine salt" contemplates salts with ammonia and organic nitrogen bases strong enough to form salts with carboxylic acids. Said pharmaceutically acceptable salts can be obtained by conventional methods known by persons skilled in the art.

As it is used herein, the term "statin" relates to an inhibitor of the 3-hydroxy-3-methylglutaryl-coenzyme A reductase (HMG-CoA) enzyme, which catalyzes the limiting step of cholesterol biosynthesis and includes any natural, synthetic or semi-synthetic statin.

Therapeutic uses of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid

In one aspect, the invention relates to a (1*S*,3*R*,7S,8*S*,8a*R*)-1,2,3,7,8,8a-hexahydro-3,7-dimethyl-8-[2-[(2*R*, 4*R*)-tetra-hydro-4-hydroxy-6-oxo-2*H*-pyran-2-yl]ethyl-1-naphthalenyl propanoate derivative, optionally mono- or di-methylated at the carbon in the alpha position of propanoic acid of formula (I)

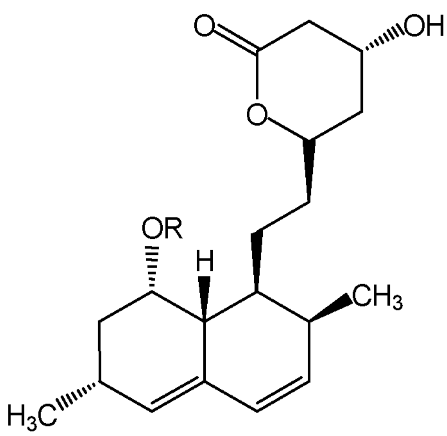

(I)

wherein R is $CH_3CH_2CO$-, $(CH_3)_2CHCO$- or $(CH_3)_3CCO$-, its hydroxy acid forms and the pharmaceutically acceptable salts of said hydroxy acids, for use in the treatment and/or prevention of:

a) neurodegenerative diseases, or
b) diseases associated with an unwanted oxidation, or
c) age-associated pathological processes, or
d) epilepsy, epileptic seizures or convulsions.

In a particular embodiment, said compound of formula (I) is the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate [compound of formula (I) wherein R is $CH_3CH_2CO$-, also occasionally identified in this description as NST0003], its hydroxy acid form or a pharmaceutically acceptable salt of said hydroxy acid.

In another particular embodiment, said compound of formula (I) is the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate [compound of formula (I) wherein R is $(CH_3)_2CHCO$-, also occasionally identified in this description as NST0004], its hydroxy acid form or a pharmaceutically acceptable salt of said hydroxy acid.

In another particular embodiment, said compound of formula (I) is the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate [compound of formula (I) wherein R is $(CH_3)_3CCO$-, also occasionally identified in this description as NST0005], its hydroxy acid form or a pharmaceutically acceptable salt of said hydroxy acid.

The invention contemplates both the use of one of said compounds of formula (I), its hydroxy acid form or a pharmaceutically acceptable salt of said hydroxy acid, and of a mixture of two or more compounds of formula (I), their hydroxy acid forms or pharmaceutically acceptable salts thereof. Thus in a particular embodiment, said compound of formula (I) is selected from the group consisting of:

- (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate,
- (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate,
- (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate,
- their hydroxy acid forms and the pharmaceutically acceptable salts of said hydroxy acids, and

- their mixtures.

The compounds of formula (I) are known compounds and can be obtained by conventional semisynthesis methods, such as those described in United States patent US 4866090, or by means of biotransformation, by means of a process comprising the production of monacolin J by fermentation from a microorganism that produces monacolin J and the subsequent acylation of the hydroxyl group present in position C8 of monacolin J by means of adding to the fermentation medium an acylating agent suitable for obtaining the desired compound of formula (I), such as an acid of general formula

$R^1COOH$ (II)

where $R^1$ is $CH_3CH_2$-, $(CH_3)_2CH$- or $(CH_3)_3C$-, or a derivative thereof selected from a halide, an ester, an amide, an anhydride or a salt of said carboxylic acid of formula (II). Illustrative examples of compounds of formula (II) include propanoic, 2-methylpropanoic, and 2,2-dimethylpropanoic acids.

A number of assays performed by the inventors have clearly shown the neuroprotective effect of the compounds of formula (I) against the action of a substance causing oxidative stress, as well as its neuroprotective effect against the action of a substance causing endoplasmic reticulum stress, and its neuroprotective effect against the action of a substance causing apoptosis in human cholinergic neurons.

The neuroprotective effect against the action of a substance causing oxidative stress, such as xanthine/xanthine oxidase (XXO), is described in Example 1. It is observed in said example that the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate (NST0003), by way of illustration, is capable of significantly inhibiting the production of reactive oxygen species (ROS) without affecting cell viability, which demonstrates a neuroprotective effect of the compounds of formula (I). A known natural antioxidant, ascorbic acid (AA), is used as control of the inhibition of ROS production.

For the purpose of better defining the neuroprotective effect of the compounds of formula (I), the inventors analyzed the neurodegenerative process in more detail by means of the analysis of neuronal death caused by oxidative stress, using XXO as the substance causing said oxidative stress which triggers cell death. The obtained results (Figure 2) illustrate that the treatment with the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005), by way of illustration, quantitatively and significantly reduces neuronal death caused by oxidative stress, which clearly shows the neuroprotective capacity of said compounds of formula (I). This neuroprotective action is independent of the inhibitory capacity of the HMG-CoA reductase enzyme (statin activity) as shown in Figure 3, where it can be seen that the inhibitory activity of the HMG-CoA reductase enzyme of the compounds of formula (I) is very far from the inhibitory activity of said HMG-CoA reductase enzyme of a known statin, atorvastatin; in fact, it can be seen in said Figure 3 that the NST0003 compound inhibits the HMG-CoA reductase enzyme 170 times less than atorvastatin, the NST0004 compound inhibits said enzyme 100 times less than atorvastatin, and the NST0005 compound inhibits the HMG-CoA reductase enzyme 4 times less than atorvastatin.

The neuroprotective effect against the action of a substance causing endoplasmic reticulum stress (tunicamycin) is described in Example 2. It is observed in said example that the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005), by way of illustration, is capable of quantitatively and significantly reducing neuronal death caused by endoplasmic reticulum stress, which clearly shows the neuroprotective capacity of said compounds of formula (I).

The neuroprotective effect against the action of a substance causing apoptosis (camptothecin) is described in Example 3. It is observed in said example that the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate (NST0004), by way of illustration, is capable of quantitatively and significantly reducing neuronal death caused by apoptosis, which clearly shows the neuroprotective capacity of said compounds of formula (I). Likewise, for the purpose of better defining the neuroprotective effect of said compounds of formula (I), the inventors analyzed the neurodegenerative process in more detail by means of flow cytometry analysis of neuronal death caused by apoptosis and its inhibition by said compounds of formula (I) in comparison with a specific inhibitor of neuronal death by apoptosis, Z-VAD-fmk. It is observed in said Example 3 that the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005), by way of illustration, is capable of quantitatively and significantly inhibiting the neuronal death caused by apoptosis.

The protective capacity of the compounds (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate (NST0003), (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate (NST0004) and (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) against cell death caused by

apoptosis in an animal model (zebrafish) is shown in Example 4. Said NST0003, NST0004 and NST0005 compounds are capable of crossing the blood-brain barrier (BBB) (Example 5). The protective effect of the NST0005 compound against degeneration, neuritic dystrophy, necrosis, apoptosis of hippocampal pyramidal neurons and astrogliosis in the cerebral cortex is shown in Examples 6 and 7.

In addition, the protective effect of the compounds of formula (I) against epileptic seizures and convulsions is clearly shown in Example 8. It is observed in said example that said NST0005 compound, by way of illustration, is capable of protecting against epileptic seizures and convulsions in an epilepsy model in mice obtained after the administration of an excitotoxic substance (kainate) to said mice (Example 8, Figures 13 and 14); it is observed in said example that the administration of said NST0005 compound reduces the number of animals that presented convulsions compared to the control group as well as the number of animals that entered in *status epilepticus,* which clearly shows the antiepileptic and anticonvulsant effect of said compounds of formula (I) and their potential use in the prevention and/or treatment of epilepsy and of convulsive seizures or convulsions.

For its administration in the prevention and/or treatment of neurodegenerative diseases, or of diseases associated with an unwanted oxidation, or of age-associated pathological processes, or of epilepsy, convulsive seizures or convulsions, the compound of formula (I) (its hydroxy acid forms and their pharmaceutically acceptable salts) will be formulated in a pharmaceutical composition, in a therapeutically effective amount, together with one or more pharmaceutically acceptable carriers or excipients.

The pharmaceutical composition provided by this invention can contain one or more compounds of formula (I), or a hydroxy acid form thereof or a pharmaceutically acceptable salt of said hydroxy acid together with one or more pharmaceutically acceptable carriers or excipients. In a particular embodiment, said pharmaceutical composition comprises a single compound of formula (I), or a hydroxy acid form thereof or a pharmaceutically acceptable salt of said hydroxy acid. In another particular embodiment, said pharmaceutical composition comprises two or more compounds of formula (I), their hydroxy acid forms or the pharmaceutically acceptable salts of said hydroxy acids. Said pharmaceutical composition is useful for the treatment of neurodegenerative diseases, or of diseases associated with an unwanted oxidation, or of age-associated pathological processes, or of epilepsy, convulsive seizures or convulsions.

The pharmaceutical compositions containing a compound of formula (I), or a hydroxy acid form thereof or a pharmaceutically acceptable salt of said hydroxy acid, can be formulated in any pharmaceutical dosage form suitable for its administration by the chosen administration route, e.g., oral, parenteral (subcutaneous, intramuscular, intravenous, intraperitoneal route, etc.), topical, rectal route, etc. By way of a non-limiting illustration, the pharmaceutical compositions provided by this invention can be formulated in a solid pharmaceutical dosage form administered by oral route (e.g., granules, tablets, capsules, etc.), in a liquid pharmaceutical dosage form administered by oral route (e.g., solutions, suspensions, emulsions, etc.), in a pharmaceutical dosage form administered by parenteral route (e.g., solutions, suspensions, emulsions, etc.). To that end, in each case, the suitable pharmaceutically acceptable carriers and excipients will be chosen for the chosen pharmaceutical dosage form and route of administration, for example, binding agents, diluents, disintegrating agents, lubricants, wetting agents, etc., for the formulation of solid pharmaceutical dosage forms, and buffers, surfactants, etc., for the formulation of liquid pharmaceutical dosage forms. Said carriers and excipients must be pharmaceutically acceptable and pharmacologically tolerable and have to be able to be combined with other components of the formulation without exerting any adverse effect on the treated subject. Information on said carriers and excipients, as well as on said pharmaceutical dosage forms of said active ingredient can be found in Galenic Pharmacy treatises. A review of the different pharmaceutical dosage forms of drugs, in general, and of their methods of preparation can be found in the book "Tratado de Farmacia Galénica", by C. Fauli i Trillo, 1st Edition, 1993, Luzán 5, S.A. de Ediciones.

The pharmaceutical composition provided by this invention comprises at least one compound of formula (I), or a hydroxy acid form thereof or a pharmaceutically acceptable salt of said hydroxy acid, in a therapeutically effective amount. In the way used in this description, the expression "therapeutically effective amount" relates to the amount of compound calculated to cause the desired effect. The dose of the compound of formula (I), or a hydroxy acid form thereof or a pharmaceutically acceptable salt of said hydroxy acid, to be administered to a subject can vary within a wide range depending on a number of factors, including the characteristics of the compound used, e.g., its biological half-life and activity, the concentration of the compound in the pharmaceutical composition, the clinical situation of the subject, the severity of the pathology, the chosen pharmaceutical dosage form, etc.. The pharmaceutical composition provided by this invention can be administered one or more times a day for preventive or therapeutic purposes or, alternatively, other administration regimens can be followed, not necessarily daily but also at precise times, weekly, etc.

If desired, the pharmaceutical composition provided by this invention can be used together with other drugs, for example, drugs useful in the treatment of neurodegenerative diseases, or diseases associated with an unwanted oxidation, or of age-associated pathological processes, or drugs useful in the treatment of epilepsy, convulsive seizures or convulsions, for the purpose of increasing the efficacy of the pharmaceutical composition provided by this invention, a combination therapy thus being generated. Said additional drugs can form part of the same pharmaceutical composition or, alternatively, can be provided as a separate pharmaceutical composition for its administration at the same time

(simultaneous administration) as the pharmaceutical composition provided by this invention or at different times (sequential administration) with respect to the administration of the pharmaceutical composition provided by this invention. Illustrative, non-limiting examples of said drugs useful in the treatment of neurodegenerative diseases, or diseases associated with an unwanted oxidation, or of age-associated pathological processes, or of epilepsy, convulsive seizures or convulsions, include acetylcholinesterase inhibitors (e.g., donepezil, galantamine, rivastigmine, etc.), NMDA glutamate receptor antagonists (e.g., memantine, etc.), antioxidants (e.g. vitamins, tocopherols, carotenoids, polyphenols, etc.), natural extracts (e.g., curcumin, *Ginkgo* biloba extract, melatonin, *Bacopa monniera* extract, etc.), antiepileptic agents (e.g., barbiturates, benzodiazepines, bromides, carbamates, hydantoinates, lamotrigine, gabapentin, etc.), anticonvulsant agents (e.g., repeatedly activated sodium channel blockers such as phenytoin, carbamazepine, oxcarbazepine, etc.; drugs enhancing the actions of the GABA neurotransmitter such as phenobarbital, benzodiazepines, etc.; glutamate modulators such as topiramate, lamotrigine, felbamate, etc.; T-type calcium channel blockers such as ethosuximide, valproic acid , etc.; N and L-type calcium channel blockers such as lamotrigine, topiramate, zonisamide, valproic acid, etc.; H flux modulators, gabapentin, lamotrigine, etc.; specific biding site blockers, such as gabapentin, levetiracetam, etc., carbonic anhydrase inhibitors such as topiramate, etc.).

In another aspect, the invention relates to the use of a compound of formula (I), or of a hydroxy acid form thereof or of a pharmaceutically acceptable salt of said hydroxy acid, in the preparation of a pharmaceutical composition for the prevention and/or treatment of:

a) neurodegenerative diseases, or
b) diseases associated with an unwanted oxidation, or
c) age-associated pathological processes, or
d) epilepsy, convulsive seizures or convulsions.

The features of the compound of formula (I), its hydroxy acid forms and the pharmaceutically acceptable salts of said hydroxy acid, as well as those of said diseases have already been mentioned above.

In another aspect, the invention relates to a method for the prevention and/or treatment of neurodegenerative diseases, or of diseases associated with an unwanted oxidation, or of age-associated pathological processes, or of epilepsy, convulsive seizures or convulsions, which comprises administering to a subject in need of treatment a therapeutically effective amount of a compound of formula (I), its hydroxy acid forms or the pharmaceutically acceptable salts of said hydroxy acid, or of a pharmaceutical composition provided by this invention comprising a compound of formula (I), a hydroxy acid form thereof or a pharmaceutically acceptable salt of said hydroxy acid; the features of the compound of formula (I), its hydroxy acid forms and the pharmaceutically acceptable salts of said hydroxy acid, as well as those of said diseases and pharmaceutical compositions comprising a compound of formula (I) have already been mentioned above.

The following examples serve to illustrate the invention and must not be considered as limiting thereof.

The compounds identified as NST0003, NST0004 and NST0005 in said examples have been prepared according to the methodology described in Hoffman, et al. (J. Med. Chem., 1986, 29, 849-852).

## EXAMPLE 1

### Inhibition of the production of reactive oxygen species by NST0003 and protection by NST0005 against neuronal death induced by oxidative stress regardless of the statin activity (inhibitory of the HMG-CoA reductase enzyme)

### 1.1 Inhibition of the production of reactive oxygen species (ROS) by NST0003

The assay was performed on human neuroblastoma SK-N-MC cells in culture from the American Type Culture Collection (ATCC) (HTB-10), in all cases strict rules of sterility were followed and the manipulation was performed in class II biological safety cabinets following European standard EN 12469. The cells were maintained in Minimum Essential Medium Eagle (MEM) culture medium supplemented with 1 mM sodium pyruvate, 2 mM L-glutamine, 0.1 mM non-essential amino acids, 0.05 mg/ml gentamicin and 10% fetal bovine serum.

The inhibition by the NST0003 compound [(1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate] on the production of reactive oxygen species (ROS) in the SK-N-MC cells, was analyzed. These cells, not exceeding 15 passages, were seeded on 96-well plates treated for adherent cells with a cell concentration of $5x10^4$ cells/well; 3 wells of the plate were seeded for each condition of the assay.

After 24 hours of cell incubation at 37˚C and 5% $CO_2$, the cell treatments were performed with 100 μl of total volume for the following conditions: (i), control (culture medium); (ii), xanthine/xanthine oxidase (XXO) [10 μM -100 mU/ml], used for producing cellular oxidative stress; (iii), XXO [10 μM -100 mU/ml] and ascorbic acid (AA) [100 μM], as

positive control of the inhibition of ROS production; and (iv), XXO [10 μM -100 mU/ml] and NST0003 at different concentrations [4, 10, 20 and 40 μg/ml].

The cells were incubated (at 37°C and 5% $CO_2$) with these treatments for 3 h, after which time they were washed with HKRB buffer (20 mM HEPES, 103 mM NaCl, 4.77 mM KCl, 0.5 mM $CaCl_2$, 1.2 mM $MgCl_2$, 1.2 mM $KH_2PO_4$, 25 mM $NaHCO_3$, 15 mM glucose; at pH 7.3) after which the 2',7'-dichlorodihydrofluorescein diacetate ($H_2$DCFDA) probe was added at 30 μM diluted in HKRB buffer and it was incubated for another 2 hours in the same conditions. The reading of the plate was taken in a fluorometer at the conditions of 485 nm for excitation and 538 nm for emission.

To correct (normalize) the results, the intracellular lactate dehydrogenase (LDH) measurement of each of the conditions was used. To perform this measurement, the cells were lysed by freezing in 100 μl of MEM, performing a prior washing with MEM. For perfect cell lysis, the plate, frozen at -20°C, was maintained at that temperature for at least 4 h. After thawing at room temperature, the medium and the lysed cells were pooled and were transferred to a 96-well V bottom plate which was centrifuged for 10 minutes at 300xg. The LDH assay was conducted on the supernatant by means of the LDH Cytotoxicity Detection Kit (Roche) following the manufacturer's specifications.

The cell viability of each condition relating to the control was thus calculated and this value served to correct the ROS production data. Figure 1 shows the percentage of corrected ROS relating to the production by XXO of each of the assayed concentrations of NST0003. Inhibition was observed at 10 and 20 μg/ml of NST0003, the maximum of 33% being at 10 μg/ml (*p<0.05, by means of Student's t test), which demonstrates a functional antioxidant effect in the cells. As the known antioxidant, AA inhibits ROS production by 80% in the assayed conditions.

Based on these results, which illustrate the decrease of ROS production by a compound of formula (I) [NST0003], the inventors then studied if said compounds of formula (I) caused a decrease of neuronal death caused by oxidative stress, and therefore, a neuroprotective effect.

1.2 <u>Protection by NST0005 against neuronal death induced by oxidative stress regardless of the statin activity</u>

To perform this assay, the inhibition caused by the NST0005 compound [(1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethyl-propanoate] of cell death caused by the treatment with xanthine/xanthine oxidase (XXO) which, as has already been mentioned, generates oxidative damage (produces free radicals such as hydrogen peroxide, superoxide anion, hydroxyl radical) which triggers cell death, was analyzed. These cells [SK-N-MC], not exceeding 15 passages, were seeded on 96-well plates treated for adherent cells with a cell concentration of $5x10^4$ cells/well; 3 wells of the plate were seeded for each condition of the assay.

After 24 h of cell incubation at 37°C and 5% $CO_2$, the cell treatments were performed with 100 μl of total volume for the following conditions: (i), control (culture medium); (ii), xanthine/xanthine oxidase (XXO) [10 μM - 60 mU/ml]; and (iii), XXO [10 μM - 60 mU/ml] and NST0005 at different concentrations [4, 10, 20 and 40 μg/ml].

The cells were incubated (at 37°C and 5% $CO_2$) with these treatments for 22 h, after which time the WST-1 reagent (Roche) was added. The WST-1 Test is based on the measurement of the metabolic activity. Cell damage causes the loss of the ability of cells to obtain the energy necessary for maintaining their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are expressed, as is shown in Figure 2, as the reduction of the percentage of cell death for each treatment relating to death caused by XXO. Protection was observed at 4, 10 and 20 μg/ml of NST0005 (*p<0.05, by means of the Student's t test), the maximum being 50% at 10 μg/ml, so the NST0005 compound shows a protective effect against the death of human neuronal cells (SK-N-MC) caused by oxidative stress.

Based on these results, the inventors decided to study if the neuroprotective capacity is dependent on the activity of the statins (i.e., inhibition of the HMG-CoA reductase enzyme). To that end, the degree of inhibition of the HMG-CoA reductase enzyme by the different (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid (NST0003, NST0004 and NST0005) was studied. The effect of these compounds was compared with that of a known statin, atorvastatin, for which a potent inhibitory effect on the HMG-CoA reductase enzyme has already been described.

During the reaction, the HMG-CoA reductase uses NADPH as a reducing agent and 3-hydroxy-3-methylglutaryl coenzyme A (HMG-CoA) as a substrate. Mevalonic acid, which serves as a substrate for the following reaction in order to continue with the cholesterol synthesis, is obtained as a reaction product. The enzymatic reaction object of study is schematically shown as follows:

$$\text{HMG-CoA} + 2\text{NADPH} + 2\text{H}^+ \rightarrow \text{Mevalonic acid} + 2\text{NADP}^+ + \text{CoASH}$$

This *in vitro* assay is based on the spectrophotometric measurement of the decrease of absorbance at 340 nm, representing the oxidation of the NADPH by the catalytic subunit of the HMG-CoA reductase in the presence of the HMG-CoA reductase substrate. The assay was carried out in a 96-well plate, with a total reaction volume of 200 μl. The reaction buffer (50 mM $KH_2PO_4$, 1 M KCl, 2 mg/ml bovine serum albumin (BSA) and 5 mM DTT, at pH=7.3) was prepared at the time of carrying out the reaction and was maintained at 37˚C. The assays included: (i), blank (without enzyme) which reports on the stability of NADPH during the reaction; (ii), control (without test compound); and (iii), test compounds: Atorvastatin, NST0003, NST0004 and NST0005 at different concentrations [0.01; 0.02; 0.04; 0.1; 0.2; 0.4; 1; 2; 4; 10; 20; 40 and 100 μg/ml] and dissolved in dimethylsulfoxide (DMSO).

Four independent assays were carried out. The test compounds were added to the reaction buffer in the plate and the concentration of DMSO was standardized by reaction at 10 μl, the non-interference thereof during the reaction having previously been demonstrated. The concentrations of the reagents involved in the reaction mixture were the following: 0.2 mM HMG-CoA, 3 μU/reaction HMG-CoA reductase and 0.2 mM NADPH. After brief agitation, the reading of the plate was taken in a spectrophotometer at the conditions of 340 nm, 37˚C, detecting the decrease of absorbance at 340 nm over time. The percentage of HMG-CoA reductase enzymatic activity with respect to the control was calculated. The $IC_{50}$ was calculated with the data obtained by means of the Trimmed Spearman-Karber method (Version 1.5).

The obtained results confirm, as is shown in Figure 3, the inhibitory effect on HMG-CoA reductase by atorvastatin ($IC_{50=}$ 0.09±0.01 μg/ml), whereas the NST0003, NST0004 and NST0005 compounds presented inhibitory activities far from the known statin: $IC_{50}$ (NST0003)= 15.30±5.05 μg/ml, $IC_{50}$ (NST0004)= 9.05±4.40 μg/ml, and $IC_{50}$ (NST0005) = 0.35±0.07 μg/ml, which indicates an inhibition power of HMG-CoA reductase of 170, 100 and 4 times less than that of atorvastatin, respectively.

EXAMPLE 2

Inhibition by NST0005 of neuronal death induced by endoplasmic reticulum stress

Based on the results previously obtained, the inventors decided to study if the (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid (NST0003, NST0004 and NST0005) were capable of protecting against cell death caused by endoplasmic reticulum stress.

To that end, an assay was performed on human neuroblastoma SK-N-MC cells in culture from the ATCC (HTB-10); in all cases strict rules of sterility were followed and the manipulation was performed in class II biological safety cabinets following European standard EN 12469. The cells were maintained in MEM culture medium supplemented with 1 mM sodium pyruvate, 2 mM L-glutamine, 0.1 mM non-essential amino acids, 0.05 mg/ml gentamicin and 10% fetal bovine serum.

The inhibition caused by the compound (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) of cell death caused by treatment with tunicamycin (Tm), which generates endoplasmic reticulum stress by inhibition of N-glycosylation of proteins, which triggers cell death, was analyzed. These cells, not exceeding 15 passages, were seeded on 96-well plates treated for adherent cells with a cell concentration of $5x10^4$ cells/well; 3 wells of the plate were seeded for each condition of the assay.

After 24 h of cell incubation at 37˚C and 5% $CO_2$ the cell treatments were performed with 100 μl of total volume for the following conditions: (i), control (culture medium); (ii), tunicamycin [20 μg/ml] (SIGMA), which causes the death of 50% of the cells; and (iii), tunicamycin [20 μg/ml] and NST0005 at different concentrations [4, 10, 20, 20 and 100 μg/ml].

The cells were incubated (at 37˚C and 5% $CO_2$) with these treatments for 22 h, after which time the WST-1 reagent (Roche) was added. The WST-1 Test, as has been previously mentioned, is based on the measurement of the metabolic activity; the metabolically active (live) cells reduce tetrazolium salt to formazan by means of the succinate-tetrazolium reductase system (of the mitochondrial respiratory chain) and the formazan which is formed can be detected colorimetrically since it has an absorbance of 440 nm. The reading was taken in a plate reader at 440 nm 2 hours after adding the reagent.

The obtained results are expressed, as is shown in Figure 4, as the reduction of the percentage of cell death for each treatment relating to death caused by Tunicamycin (Tm). Protection was observed at 4, 10, 20 and 40 μg/ml of NST0005 (*$p<0.05$, by means of the Student's t test), the maximum being 38% at 20 μg/ml, so the NST0005 compound shows a protective effect against the death of human neuronal cells caused by endoplasmic reticulum stress.

EXAMPLE 3

Inhibition by NST0004 and NST0005 of neuronal death induced by apoptosis

Based on the results previously obtained, the inventors decided to study if the (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid (NST0003, NST0004 and NST0005) were capable of protecting against cell death caused by apoptosis.

3.1 Determination of the metabolically active cells by means of the WST-1 Test (Roche) [formation of formazan]

To that end, an assay was performed on human neuroblastoma SK-N-MC cells in culture from the ATCC (HTB-10); in all cases strict rules of sterility were followed and the manipulation was performed in class II biological safety cabinets following European standard EN 12469. The cells were maintained in MEM culture medium supplemented with 1 mM sodium pyruvate, 2 mM L-glutamine, 0.1 mM non-essential amino acids, 0.05 mg/ml gentamicin and 10% fetal bovine serum.

The inhibition caused by the compound (1*S*,3*R*,7S,8*S*,8a*R*)-1,2,3,7,8,8a-hexahydro-3,7-dimethyl-8-[2-[(2*R*, 4*R*)-tetrahydro-4-hydroxy-6-oxo-2*H*-pyran-2-yl]ethyl-1-naphthalenyl 2-methylpropanoate (NST0004) of cell death caused by treatment with camptothecin (Campt), which inhibits the topoisomerase I enzyme by preventing duplication of the DNA, which triggers apoptic cell death, was analyzed. These cells, not exceeding 15 passages, were seeded on 96-well plates treated for adherent cells with a cell concentration of $5 \times 10^4$ cells well; 3 wells of the plate were seeded for each condition of the assay.

After 24 h of cell incubation at 37˚C and 5% $CO_2$ the cell treatments were performed with 100 μl of total volume for the following conditions: (i), control (culture medium); (ii), camptothecin [20 nM] (SIGMA), which causes the death of 50% of the cells; and (iii) camptothecin [20 nM] and NST0004 at different concentrations [4, 10, 20, 40 and 100 μg/ml].

The cells were incubated (at 37˚C and 5% $CO_2$) with these treatments for 22 h, after which time the WST-1 reagent (Roche) was added. The WST-1 Test is based on the measurement of the metabolic activity. The metabolically active (live) cells reduce tetrazolium salt to formazan by means of the succinate-tetrazolium reductase system (of the mitochondrial respiratory chain) and the formazan which is formed can be detected colorimetrically since it has an absorbance of 440 nm. The reading was taken in a plate reader at 440 nm 2 hours after adding the reagent.

The obtained results are expressed, as is shown in Figure 5, as the reduction of the percentage of cell death for each treatment relating to the death caused by Camptothecin (Campt). Protection was observed at 4, 10 and 40 μg/ml of NST0004 (*$p<0.05$, by means of the Student's t test), the maximum being 43% at 10 μg/ml, so the NST0004 compound shows a protective effect against the death of human neuronal cells caused by apoptosis.

Based on these results the inventors decided to study if the NST0003, NST0004 and NST0005 compounds were capable of protecting against cell death caused by apoptosis measured by flow cytometry.

3.2 Determination of the inhibition of apoptosis by means of flow cytometry

To that end, the inhibition caused by the NST0005 compound of apoptosis caused by the treatment with camptothecin (Campt) was analyzed. The SK-N-MC cells, not exceeding 15 passages, were seeded on 6-well plates treated for adherent cells with a cell concentration of $8 \times 10^5$ cells/well; 2 wells of the plate were seeded for each condition of the assay.

After 24 h of cell incubation at 37˚C and 5% $CO_2$ the cell treatments were performed with 2 ml of total volume for the following conditions: (i), control (culture medium); (ii), camptothecin [50 μM]; (iii) camptothecin [50 μM] and Z-VAD-fmk [50 μM] (Alexis), as positive control of inhibition; and (iv), 50 μM camptothecin with NST0005 at different concentrations [4, 10, 40 and 100 μg/ml].

The cells were incubated (at 37˚C and 5% $CO_2$) with these treatments for 6 h, after which time they were pooled together with their culture medium and centrifuged at 300xg for 5 minutes. The medium was removed, a washing was performed with phosphate buffered saline (PBS) and they were fixed for 2 minutes with 500 μl of 70% ethanol at -20˚C. Once fixed, they were centrifuged at 400xg for 5 minutes, washed with PBS and propidium iodine was added at 0.05 mg/ml, diluted in cycle buffer (0.1% sodium citrate, 0.3% Nonidet P-40 and 0.02 mg/ml RNAse) and incubated for 1 hour at 37˚C. After this time, they were analyzed by flow cytometry, comparing the fluorescence of the propidium iodine against the amount of DNA. The percentage of apoptosis was measured on the sub-G1 region of each of the conditions.

The obtained results are expressed, as is shown in Figure 6, as the percentage of the inhibition of the apoptosis of each treatment relating to apoptosis caused by camptothecin. Maximum protection of 17% was observed at a concentration of 4 μg/ml of NST0005 (*$p<0.05$, by means of the Student's t test), so this compound shows a protective effect against apoptosis in human neuronal cells. Z-VAD-fmk, a specific caspase inhibitor, inhibits the apoptosis caused

by 30%.

3.3 Determination of the inhibition of apoptosis by means of the measurement of caspase 3 and 7 (caspase 3/7) activation

To that end, the inhibition caused by (1*S*,3*R*,7S,8*S*,8a*R*)-1,2,3,7,8,8a-hexahydro-3,7-dimethyl-8-[2-[(2*R*, 4*R*)-tetrahydro-4-hydroxy-6-oxo-2*H*-pyran-2-yl]ethyl-1-naphthalenyl 2,2-dimethylpropanoate (NST0005) of apoptosis caused by the treatment with camptothecin (Campt) on the SK-N-MC cells in culture was analyzed following the working conditions specified in section 3.1. Apoptosis can be quantified by measuring effector caspase activation. Caspases (proteins belonging to the cysteine-protease group) mediate the cleavage of a series of proteins triggering the signaling cascade which leads cells to apoptosis. To measure active cellular caspase 3/7, a fluorometric detection kit (Apo-ONE® Homogeneous Caspase-3/7, Promega) was used. This kit is formed by a lysis buffer which lyses and makes permeable the cells in culture and by the substrate rhodamine 110,bis-(N-CBZL-aspartyl-L-glutamyl-L-valyl-L-aspartic acid amide; 2-DEVD-R110) of the effector caspases 3 and 7. The active cellular caspases 3 or 7 cause the cleavage of the substrate, emitting fluorescence which is read by means of a fluorometer.

The effect of the pretreatment of the NST0005 compound at 40 and 100 μg/ml on caspase 3/7 activation caused by 50 μM of camptothecin (Campt) in the SK-N-MC cells was thus analyzed. These cells, not exceeding 15 passages, were seeded on 96-well plates treated for adherent cells with a cell concentration of $5x10^4$ cells/well, 3 wells of the plate were seeded for each condition of the assay. After 24 h of cell incubation at 37˚C and 5% $CO_2$ the pretreatment was performed with NST0005 at 40 μg/ml and 100 μg/ml. After 24 h of pretreatment the cell treatments were performed for the following conditions: (i), control (culture medium); (ii), camptothecin [50 μM], for the groups pretreated with medium alone or with NST0005 at 40 μg/ml and 100 μg/ml; and (iii) camptothecin [50 μM] and Z-VAD-fmk [50 μM] (Alexis).

The cells were incubated (at 37˚C and 5% $CO_2$) with these treatments for 6 h, after which time the lysis buffer and the substrate rhodamine 110 were added at the concentrations specified by the manufacturer; they were incubated at room temperature for 30 minutes and the cells were then frozen at - 20˚C overnight. They were thawed the next day and the reading of the plate was taken in a fluorometer at 499 nm for excitation and 521 nm for emission.

Figure 7 shows the percentage of caspase 3/7 activation with respect to the activity obtained with the pro-apoptotic treatment with Campt in a standard assay. The Z-VAD-fmk caspase inhibitor completely inhibited caspase 3/7 activation. In turn, NST0005 caused a statistically significant inhibition of caspase 3/7 activation of 33% at the concentration of 100 μg/ml (*$p<0.05$, by means of the Student's t test), which demonstrates its protective effect against apoptosis.

EXAMPLE 4

Inhibition by the NST0003, NST0004 and NST0005 compounds of the apoptosis in the brain of the zebrafish embryo

Based on the results previously obtained, the inventors decided to study if the (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid (NST0003, NST0004 and NST0005) were capable of protecting against cell death caused by apoptosis in an animal model, the zebrafish.

To that end, an assay was performed on zebrafish larvae (96 hours post-fertilization). The fertilized eggs were obtained by a natural birth of adult zebrafish (AB strain) in the inventors' laboratory, carried out according to what is described in The *Zebrafish Book* [Westerfield, M. (1995) The Zebrafish Book. A Guide for the Laboratory Use of Zebrafish (Danio rerio). 3rd ed., University of Oregon Press, Eugene]. The eggs were collected in the $1^{st}$ hour after spawning, washed and deposited in a clean Petri dish with embryo water (medium) (4.9 mM NaCl, 0.17 mM KCl, 0.33 mM $CaCl·2H_2O$, 0.33.mM $MgSO4·7H_2O$, pH 7.2) prepared following the guidelines described in the book *Zebrafish. Practical approach* [Nusslein-Volhard, C and Dahm, R (2002) Zebrafish. Practical approach. Oxford: Oxford University Press].

The inhibition caused by the NST0003, NST0004 and NST0005 compounds on cell death caused by the tert-butyl hydroperoxide (TBH) compound, which is a pro-oxidizing compound which induces specific neurotoxicity in zebrafish larvae by means of the mechanism of apoptosis, was analyzed.

The larvae (at 96 hours post-fertilization [hpf]) were seeded in 96-well plates (3 larvae/well), performing a triplicate (3 wells) for experimental condition, thus obtaining an intra-assay triplicate. The larvae were treated in a total volume of embryo medium of 100 μl per well for the following conditions: (i) control (embryo medium); (ii), TBH [0,45 mM], which corresponds with the maximum tolerated dose (MTD), which induces neurotoxicity in the larvae but not mortality; and (iii) TBH [0.45 mM] and NST0003, NST0004 and NST0005, all at the concentration of 1 μg/ml.

The larvae were incubated with the treatments for 24 h; after this time, they were processed by means of staining with Acridine Orange (AO) [3,6-bis(dimethylamino)acridinium hydrochloride · zinc chloride, double salt (Sigma-Aldrich)] to determine the percentage of apoptosis induced by the neurotoxin. In the whole animal, AO accumulates in acid granules which are mainly formed in cells experiencing apoptosis. It has been demonstrated that this staining with

AO is capable of distinguishing between cells presenting apoptosis from necrotic cells (it does not stain them) in live embryos of *Drosophila* and furthermore it is a technique commonly used to identify apoptotic cells in whole zebrafish.

Thus, the control larvae and the treated larvae were immersed in 1 μg/ml of AO in embryo water at 28.5°C for 60 minutes. After this time, the AO was removed by means of washings with embryo water, performing a total of 5 washings, 10 minutes each. AO is a fluorescent compound which can be readily measured and quantified with a fluorometer. Therefore, the larvae were anesthetized with tricaine (3-aminobenzoic acid ethyl ester) and homogenized for their subsequent reading in a fluorescence plate reader (excitation: 488 nm; emission: 530 nm).

The obtained results are expressed, as is shown in Figure 8, as the percentage of apoptosis for each treatment relating to death caused by TBH. Neuroprotection was observed with the 3 compounds tested at the concentration of 1 μg/ml: the NST0003 compound reduced apoptosis induced by the neurotoxin by 50.7 ± 17.1%, this protection being statistically significant (*p<0.05, by means of the Student's t test)); the NST0004 compound also was capable of reducing the apoptosis caused by TBH, reducing apoptosis caused by the damage inducing compound (TBH) by 45.3 ± 18.9%, this reduction also being significant (*p<0.05, by means of the Student's t test); and the NST0005 compound also reduced apoptosis by a high percentage, causing an inhibition of 34.3 ± 17.3% of the apoptosis induced by the TBH, although despite the high inhibition, it was not statistically significant.

EXAMPLE 5

Crossing of the blood-brain barrier of NST0003, NST0004 and NST0005 by means of *in vitro* assay (PAMPA) and calculation of the theoretical lipophilicity

The purpose of the assay was to predict if the (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate derivatives, optionally mono- or di-methylated at the carbon in the alpha position of propanoic acid (NST0003, NST0004 and NST0005) were capable of crossing the blood-brain barrier (BBB). To that end, the PAMPA (Parallel Artificial Membrane Permeation Assay) assay was used, which uses a sandwich system contemplating the crossing of compounds by means of passive diffusion, preventing active transport. Verapamil, a compound with high BBB permeability, was used as positive control, whereas theophylline, a compound which does not cross the barrier, was used as negative control.

A mixture of commercial lipids derived from pig brain with a phospholipid composition very similar to that which forms the human BBB, called PBL (Porcine Polar Brain Lipid), was used to mimic the BBB, and it was stored at -20°C dissolved in dodecane at 100 μg/ml in glass vials. The verapamil and theophylline were prepared at 10 mM in DMSO, whereas the acid forms of NST0003, NST0004 and NST0005 were prepared in water and were activated with 0.1 N NaOH at 4°C for 12h.

At the time of the assay, 1.5 ml of the compounds to be evaluated at 100 μM were prepared in a phosphate buffer at pH 7.4, maintaining a constant concentration of DMSO of 1%. 5 μl of PBL at 20 μg/ml were added in a 96-well filter plate, with a polyvinylidene fluoride (PVDF) membrane, with a pore size of 45 μm (MAIPN4550, Millipore). After 2 minutes, 300 μl of the phosphate buffer used to dilute the compounds were added. This plate was considered the "acceptor" plate and was placed in the top part of the sandwich. 300 μl of the compounds were added at 100 μM on another 96-well plate which is assembled with the previous one (MATRNP550 of Millipore) (called "donor") and in triplicate. Furthermore, a blank was included with 1% DMSO in the phosphate buffer used. The acceptor plate was carefully placed on the donor plate forming the sandwich system. The compounds object of study spread from the wells of the donor plate to the corresponding wells of the acceptor plate for 18 h in which the system remained intact. The remaining compound prepared was preserved in the same conditions of humidity, temperature and darkness as the sandwich system formed by the plates. After that time, 100 μl of the wells of the donor plates and of the acceptor plates were transferred to a special 96-well plate for ultraviolet (UV) reading. Furthermore, 100 μl of the compounds prepared for performing the assay and stored in the same manner were transferred in triplicate to the plates (basal wells). The plate was introduced in a spectrophotometer in which a scan was carried out in UV from 230 to 498 nm, with readings every 4 nm. From the data provided by the spectrophotometer the percentage of barrier crossing as well as the effective permeability ($P_e$) were calculated by applying the following formula:

$$Pe = C \times -Ln\left(1- \frac{[Drug]_{acceptor}}{[Drug]_{equilibrium}}\right)$$

where:

$$C = \frac{V_D \times V_A}{(V_D \times V_A) \times Area \times Time}$$

[Drug] $_{acceptor}$ = Absorbance of the acceptor well
[Drug] $_{equilibrium}$ = Absorbance of the mean of the basal wells / 2
$V_A$= Volume of the acceptor well = 0.3 $cm^3$
$V_D$= Volume of the donor well = 0.3 $cm^3$
Area = 0.24 $cm^2$
Time = 64,000 s

A theoretical calculation of the lipophilicity of the NST0003, NST0004 and NST0005 compounds was additionally performed by means of determining the logarithm of the octanol/water partition coefficient (cLogP) by means of the CLOGP program (OSIRIS Property Explorer) by entering the chemical structures in the software.

Figure 9 shows the results of the BBB crossing calculated by means of the PAMPA method (expressed as $P_e$) in correlation with the percentage of BBB crossing. Additionally, the attached table (Figure 9) shows the cLogP theoretical lipophilicity index. Out of the three derivatives, NST0005 is the compound with the highest capacity to cross the BBB, which is correlated with its higher lipophilicity.

EXAMPLE 6

Protection by NST0005 against degeneration, neuritic dystrophy, necrosis and apoptosis of the hippocampal pyramidal neurons of mice brains

6.1 Protective effect of NST0005 against neurodegeneration in the hippocampus of mice caused by an excitotoxic substance

Based on the previous results, the inventors decided to investigate if the neuroprotective effect of (1*S*,3*R*,7S, 8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) demonstrated in human cholinergic neurons was corroborated in a model of sporadic Alzheimer's disease in mice by means of the administration of kainate (KA).

All the animals included during the experimental process were 12-week old males of the FVB/NHan strain. The experiments were carried out strictly following the Guidance on the Operation of Animals (Scientific Procedures, Act. 1986). The animals had their respective quarantine period and were treated with maximum precaution to minimize possible contaminations for inoculations and handling.

Sixteen animals were used in this assay and the treatments were all carried out by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the next 7 days with a daily dose of PBS.
ii) PBS+KA+PBS regimen: 6 animals were initially treated with PBS with a daily dose for 2 days, on the second day the animals were inoculated with a dose of kainate at 25 mg/kg and for the next 7 days with a daily dose of PBS.
iii) NST0005+KA+NST0005 regimen: 6 animals were initially treated with NST0005 with a daily dose of 50 mg/kg for 2 days, on the second day the animals were inoculated with a dose of kainate at 25 mg/kg and for the next 7 days with a daily dose of NST0005 at 50 mg/kg.

Once the treatment time of 7 days ended, the animals were sacrificed and the brains were dissected. The brain samples were processed and included in paraffin. Fluoro-Jade B® (FJB) fluorescent stain was used in 5 μm thick sections to analyze the neurodegeneration of the hippocampus.

As shown in Figure 10, row A, the PBS+KA+PBS administration regimen produced an increase of the FJB positive neurons in the CA2 and CA3 regions of the hippocampus with respect to the PBS+PBS+PBS administration

regimen. However, the samples of the NST0005+KA+NST0005 group presented a staining pattern similar to the controls (PBS+PBS+PBS group) without evidence of neurodegeneration in the CA2 and CA3 regions.

In summary, the pretreatment with NST0005 (NST0005+KA+NST0005 group) completely protected against the neurodegeneration caused by KA in the hippocampus.

6.2 <u>Protective effect of NST0005 against neuritic dystrophy in the hippocampus of mice caused by an excitotoxic substance</u>

Based on the neuroprotection results shown in the previous section, the inventors decided to investigate if the neuroprotective effect of NST0005 in the hippocampus of mice against the administration of kainate (KA) was accompanied by the protection of another sign of neuronal damage such as neuritic dystrophy. To that end, the same experimental scheme was used as in section 6.1. Once the treatment period of 7 days ended, the animals were sacrificed and the brains were dissected. The brain samples were processed and included in paraffin. Bright field immunohistochemistry of the microtubule-associated protein 2 (MAP2) was used in 5 $\mu$m thick sections to analyze the neuritic dystrophy of the hippocampus.

As shown in Figure 10, row B, the PBS+KA+PBS administration regimen produced a decrease of the MAP2 labeling, especially in CA1 areas of the hippocampus, with respect to the PBS+PBS+PBS administration regimen which shows a uniform labeling. In turn, the samples of the NST0005+KA+NST0005 group presented a staining pattern similar to the controls (PBS+PBS+PBS group) without evidence of neurodegeneration in the CA2 and CA3 regions.

In summary, the pretreatment with NST0005 (NST0005+KA+NST0005 group) protects against neurodegeneration and significantly prevents the neuritic dystrophy caused by KA in the hippocampus.

6.3 <u>Protective effect of NST0005 against the apoptosis in the hippocampus of mice caused by an excitotoxic substance</u>

Based on the neuroprotection results shown in the previous sections, the inventors decided to investigate if the neuroprotective effect of NST0005 in the hippocampus of mice against the administration of kainate (KA) was accompanied by the inhibition of neuronal death by apoptosis. To that end, the same experimental scheme was used as in section 6.1. Once the treatment period of 7 days ended, the animals were sacrificed and the brains were dissected. The brain samples were processed and included in paraffin. Acridine Orange (AO) fluorescent stain was used in 5 $\mu$m thick sections to analyze the apoptosis in the hippocampus.

As shown in Figure 10, row C, the PBS+KA+PBS administration regimen produced an increase of the number of neurons positive for AO in the CA3 region of the hippocampus with respect to the PBS+PBS+PBS administration regimen. Surprisingly, the samples of the NST0005+KA+NST0005 group presented a staining pattern similar to the controls (PBS+PBS+PBS group) with a drastic reduction of the neurons positive for AO in the CA3 region.

In summary, the pretreatment with NST0005 (NST0005+KA+NST0005 group) prevents neuronal damage, neurodegeneration and also neuronal death by apoptosis caused by KA in the hippocampus.

6.4 <u>Protective effect of NST0005 against the necrosis caused by an excitotoxic substance in the hippocampus of mice</u>

Based on the neuroprotection results shown in the previous sections, the inventors decided to investigate if the neuroprotective effect of NST0005 in the hippocampus of mice against the administration of kainate (KA) was accompanied by the inhibition of neuronal death by necrosis. To that end the same experimental scheme was used as in section 6.1. Once the treatment period of 7 days ended, the animals were sacrificed and the brains were dissected. The brain samples were processed and included in paraffin. Hematoxylin and eosin stain was used in 5 $\mu$m thick sections to analyze the necrosis in the hippocampus.

As shown in Figure 10, row D, the PBS+KA+PBS administration regimen induces a destructuring of the cell architecture of the hippocampus with numerous pyknotic nuclei in the CA3 region, in comparison with the PBS+PBS+PBS administration regimen which shows an intact structure of the pyramidal neurons of this region of the hippocampus. The samples of the NST0005+KA+NST0005 group present a staining pattern similar to the controls (PBS+PBS+PBS group) maintaining the structure of the pyramidal neurons of the CA3 region.

In summary, the pretreatment with NST0005 (NST0005+KA+NST0005 group) substantially prevents neuronal death by necrosis caused by KA in the hippocampus.

EXAMPLE 7

Protection by NST0005 against neuritic dystrophy and against astrogliosis in the cerebral cortex of mice

7.1 Protective effect of NST0005 against neuronal damage by neuritic dystrophy in the cerebral cortex of mice caused by an excitotoxic substance

Based on the results of Example 6, the inventors decided to investigate if the neuroprotective effect of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) demonstrated in neurons of the hippocampus of mice by means of the administration of kainate (KA) extended to other regions of the brain involved in Alzheimer's disease, such as the lateral entorhinal and amygdaloid nuclei of the cerebral cortex.

All the animals included during the experimental process were 12-week old males of the FVB/NHan strain. The experiments were carried out strictly following the Guidance on the Operation of Animals (Scientific Procedures, Act. 1986). The animals had their respective quarantine period and were treated with maximum precaution to minimize possible contaminations for inoculations and handling.

Sixteen animals were used in this assay and the treatments were all carried out by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the next 7 days with a daily dose of PBS.

ii) PBS+KA+PBS regimen: 6 animals were initially treated with PBS with a daily dose for 2 days, on the second day the animals were inoculated with a dose of kainate at 25 mg/kg and for the next 7 days with a daily dose of PBS.

iii) NST0005+KA+NST0005 regimen: 6 animals were initially treated with NST0005 with a daily dose of 50 mg/kg for 2 days, on the second day the animals were inoculated with a dose of kainate at 25 mg/kg and for the next 7 days with a daily dose of NST0005 at 50 mg/kg.

Once the treatment period of 7 days ended, the animals were sacrificed and the brains were dissected. The brain samples were processed and included in paraffin. Bright field immunohistochemistry of the microtubule-associated protein 2 (MAP2) was used in 5 μm thick sections to analyze the neuritic dystrophy of the cerebral cortex.

As shown in Figure 11, the PBS+KA+PBS administration regimen produces a decrease of the MAP2 labeling both in the lateral entorhinal nucleus and in the amygdaloid nucleus of the cortex, in comparison with the PBS+PBS+PBS administration regimen which shows a uniform labeling. However, the samples of the NST0005+KA+NST0005 group show a much lower loss of the MAP2 labeling than the PBS+KA+PBS group in these two regions of the limbic system.

In summary, the pretreatment with NST0005 (NST0005+KA+NST0005 group) significantly reduces neuronal damage by neuritic dystrophy caused by KA in the cerebral cortex.

7.2 Protective effect of NST0005 against astrogliosis in the cerebral cortex of mice caused by an excitotoxic substance

Based on the neuroprotection results shown in the previous section, the inventors decided to investigate if the neuroprotective effect of NST0005 in the cerebral cortex of mice against the administration of kainate (KA) was accompanied by the inhibition of a decrease of the reactive astrogliosis which is induced as a consequence of neuronal damage. To that end, the same experimental scheme was used as in section 7.1. Once the treatment period of 7 days ended, the animals were sacrificed and the brains were dissected. The brain samples were processed and included in paraffin. Bright field immunohistochemistry of the glial fibrillary acidic protein (GFAP) was used in 5 μm thick sections to analyze the reactive astrogliosis in the cerebral cortex.

As shown in Figure 12, the PBS+KA+PBS administration regimen produces astrocyte activation and proliferation in the lateral entorhinal and amygdaloid nuclei of the cerebral cortex, whereas the PBS+PBS+PBS administration regimen does not show astrogliosis in said areas. However, the samples of the NST0005+KA+NST0005 group presented a labeling pattern for GFAP similar to the controls (PBS+PBS+PBS group) without evidence of astrocyte activation or proliferation in these regions of the cerebral cortex.

In summary, the pretreatment with NST0005 (NST0005+KA+NST0005 group) significantly reduces neuronal damage and significantly prevents reactive astrogliosis caused by KA in the cerebral cortex.

EXAMPLE 8

Antiepileptic effect of NST0005 against the action of an excitotoxic substance administered to mice

It is known that the administration of an excitotoxic substance to animals induces, in some cases, epileptic seizures and convulsions. As a result the inventors decided to investigate if the neuroprotective effect of (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate (NST0005) was accompanied by an antiepileptic effect caused by an excitotoxic substance.

All the animals included during the experimental process were 12-week old males of the FVB/NHan strain. The experiments were carried out strictly following the Guidance on the Operation of Animals (Scientific Procedures, Act. 1986). The animals had their respective quarantine period and were treated with maximum precaution to minimize possible contaminations for inoculations and handling.

The animals were inoculated intraperitoneally with 25 mg/kg of kainate (KA) dissolved in PBS. Nine animals were pretreated at 24 and 0.5 hours before the inoculation with KA by means of intraperitoneal injection with PBS (PBS+KA administration regimen) and 9 were pretreated at 24 and 0.5 hours before the inoculation with KA by means of intraperitoneal injection with NST0005 at a dose of 50 mg/kg (NST0005+KA administration regimen). After the inoculation, the animals were individually housed in trays to monitor them. The maximum epilepsy level of the animals according to Racine's scale was recorded during the observation every ten minutes and for at least 120 minutes post-inoculation (m.p.i.).

Comparative studies of the epileptogenic phenomena between the treatment with PBS (PBS+KA) and with NST0005 (NST0005+KA) were subsequently conducted, the data of which are shown in Table 1.

Table 1

| | Animals with convulsions (%) | Animals with *status epilepticus* (%) | Mortality (%) |
|---|---|---|---|
| PBS+KA+PBS | 66.7 | 66.7 | 33.3 |
| NST0005+KA+NST0005 | 33.3 | 22.2 | 11.1 |

Surprisingly, differences were observed in the percentage of animals that presented convulsions among the NST0005+KA group, which was 33.3% (3 out of 9), compared to the PBS+KA treatment regimen group, which was 66.7% (6 out of 9), which demonstrates that the NST0005 compound is antiepileptic and anticonvulsant. Furthermore, differences were also observed in the percentage of animals that entered in *status epilepticus* (defined as tonic-clonic seizures for at least 30 minutes without interruption), among the NST0005+KA group, which was 22.2% (2 out of 9), compared to the PBS+KA treatment regimen group, which was 66.7% (6 out of 9), which demonstrates that the NST0005 compound is antiepileptic. This antiepileptic effect also protects against the death induced by convulsive episodes since the NST0005+KA group presented a mortality of 11.1% (1 out of 9) whereas in the PBS+KA group it was 33.3% (3 out of 9).

To corroborate these results, the latency period (defined as the time in which the first convulsions appear) among the different groups was studied, and it was observed that the latency of the NST0005+KA group (108.9±5.9 min) was greater in a statistically significant manner (*p<0.05, by means of the Student's t test) with respect to the PBS+KA treatment regimen (60.6±15.6 min) as is shown in Figure 13, which confirms the antiepileptic and anticonvulsant effect of the NST0005 compound.

Based on these results, the inventors decided to investigate if the antiepileptic and anticonvulsant effect was correlated with the epilepsy levels and with the severity of the symptoms. It was thus observed that the animals of the group with the PBS+KA regimen exceeded epilepsy level 4 in Racine's scale, whereas the mice with the NST0005+KA regimen did not reach epilepsy level 3 at any time. Thus, and as is shown in Figure 14, the onset kinetics of the epileptogenic symptoms caused by KA are different in a statistically significant manner between the treatment with NST0005 and with PBS ($F_{(1,16)}$ = 4,72; *p< 0.05; general analysis of variance by means of the ANOVA test with a Newman-Keuls post-hoc test). These results confirm the antiepileptic and anticonvulsant effect of the NST0005 compound against the administration of an excitotoxic substance.

## Claims

**1.** A compound of formula (I)

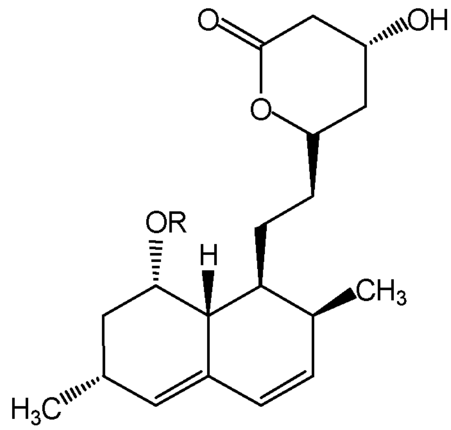

(I)

wherein R is $CH_3CH_2CO$-, $(CH_3)_2CHCO$- or $(CH_3)_3CCO$-, its hydroxy acid forms and the pharmaceutically acceptable salts of said hydroxy acids, for use in the prevention and/or treatment of:

a) neurodegenerative diseases, or
b) diseases associated with an unwanted oxidation, or
c) age-associated pathological processes, or
d) epilepsy, convulsive seizures or convulsions.

**2.** The compound according to claim 1, selected from (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate, its hydroxy acid form or a pharmaceutically acceptable salt of said hydroxy acid.

**3.** The compound according to claim 1, selected from (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate, its hydroxy acid form or a pharmaceutically acceptable salt of said hydroxy acid.

**4.** The compound according to claim 1, selected from (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate, its hydroxy acid form or a pharmaceutically acceptable salt of said hydroxy acid.

**5.** The compound according to claim 1, selected from the group consisting of:

- (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl propanoate,
- (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2-methylpropanoate,
- (1*S*,3*R*,7S,8*S*,8a*R*)-1,2,3,7,8,8a-hexahydro-3,7-dimethyl-8-[2-[(2*R*,4*R*)-tetrahydro-4-hydroxy-6-oxo-2*H*-pyran-2-yl]ethyl-1-naphthalenyl 2,2-dimethylpropanoate,
- their hydroxy acid forms and the pharmaceutically acceptable salts of said hydroxy acids, and
- their mixtures.

**6.** Use of a compound of formula (I)

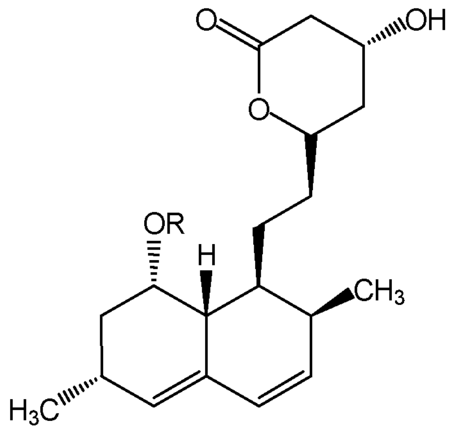

(I)

wherein R is $CH_3CH_2CO$-, $(CH_3)_2CHCO$- or $(CH_3)_3CCO$-, its hydroxy acid forms and the pharmaceutically acceptable salts of said hydroxy acids, in the preparation of a pharmaceutical composition for the prevention and/or treatment of:

a) neurodegenerative diseases, or
b) diseases associated with an unwanted oxidation, or
c) age-associated pathological processes, or
d) epilepsy, convulsive seizures or convulsions.

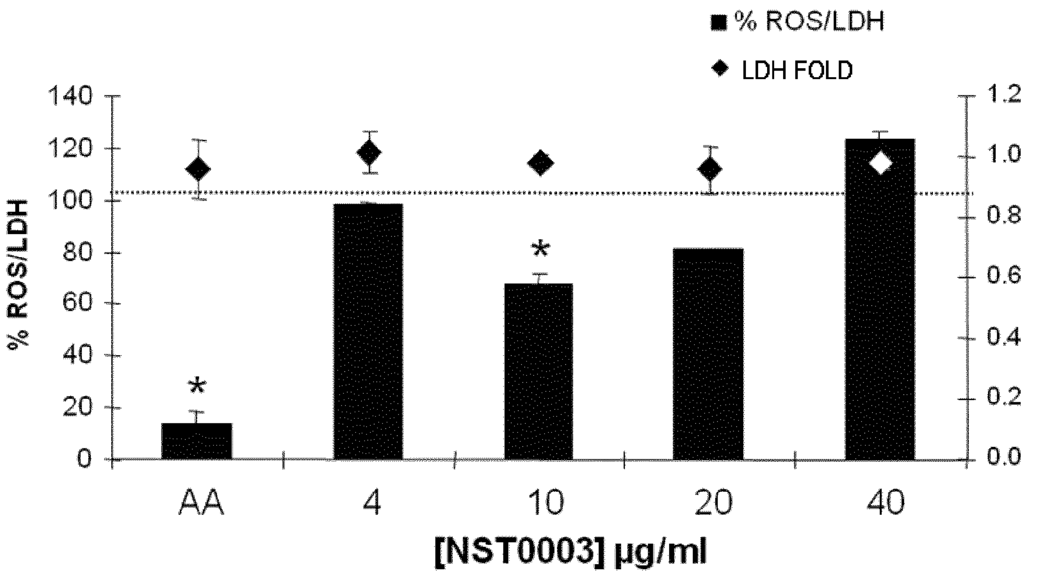
% ROS/LDH
LDH FOLD
140
120
100
80
60
40
20
0
% ROS/LDH
1.2
1.0
0.8
0.6
0.4
0.2
0.0
LDH FOLD
*
*
AA
4
10
20
40
[NST0003] µg/ml

Figure 1

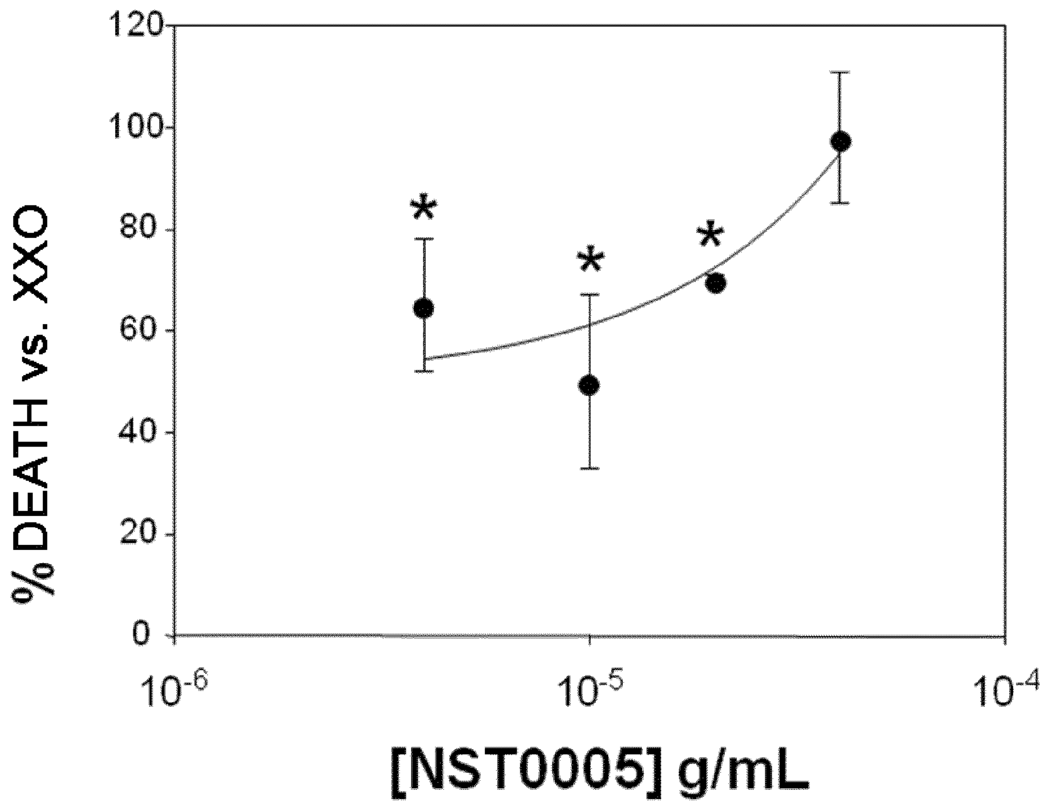


Figure 2

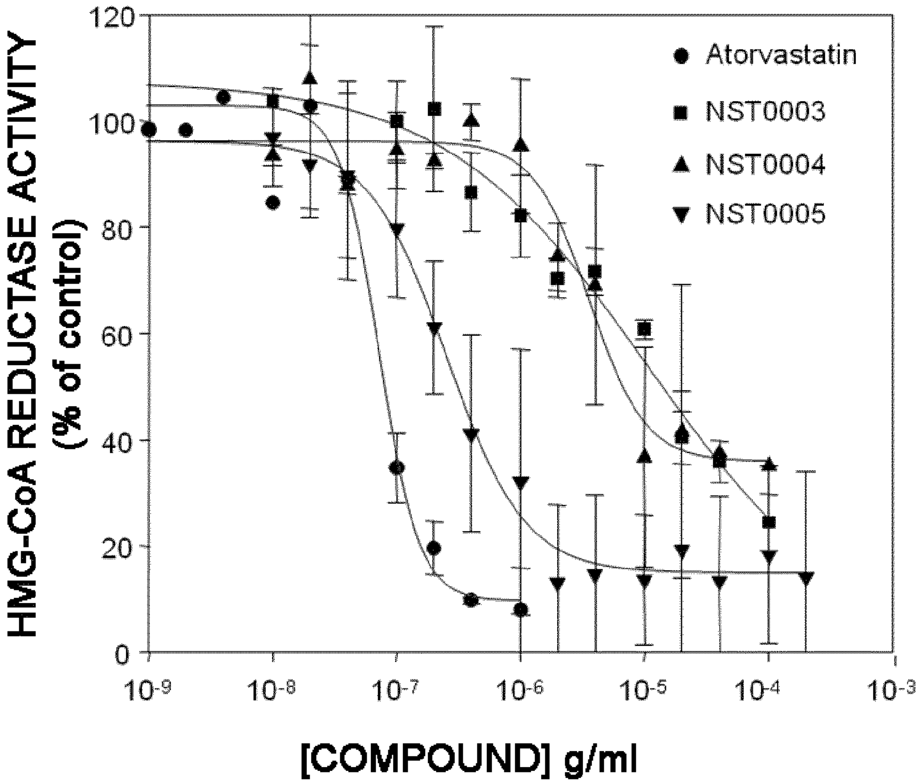
HMG-CoA REDUCTASE ACTIVITY
(% of control)
120
100
80
60
40
20
0
Atorvastatin
NST0003
NST0004
NST0005
$10^{-9}$
$10^{-8}$
$10^{-7}$
$10^{-6}$
$10^{-5}$
$10^{-4}$
$10^{-3}$
[COMPOUND] g/ml

Figure 3

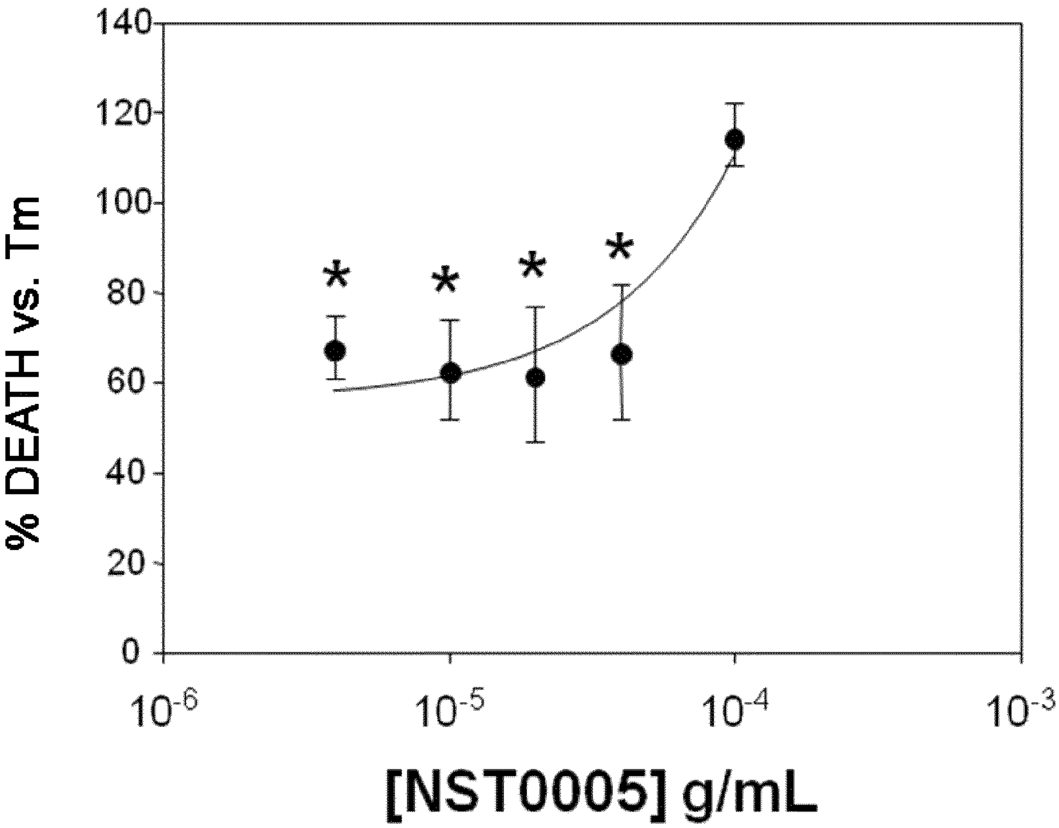


Figure 4

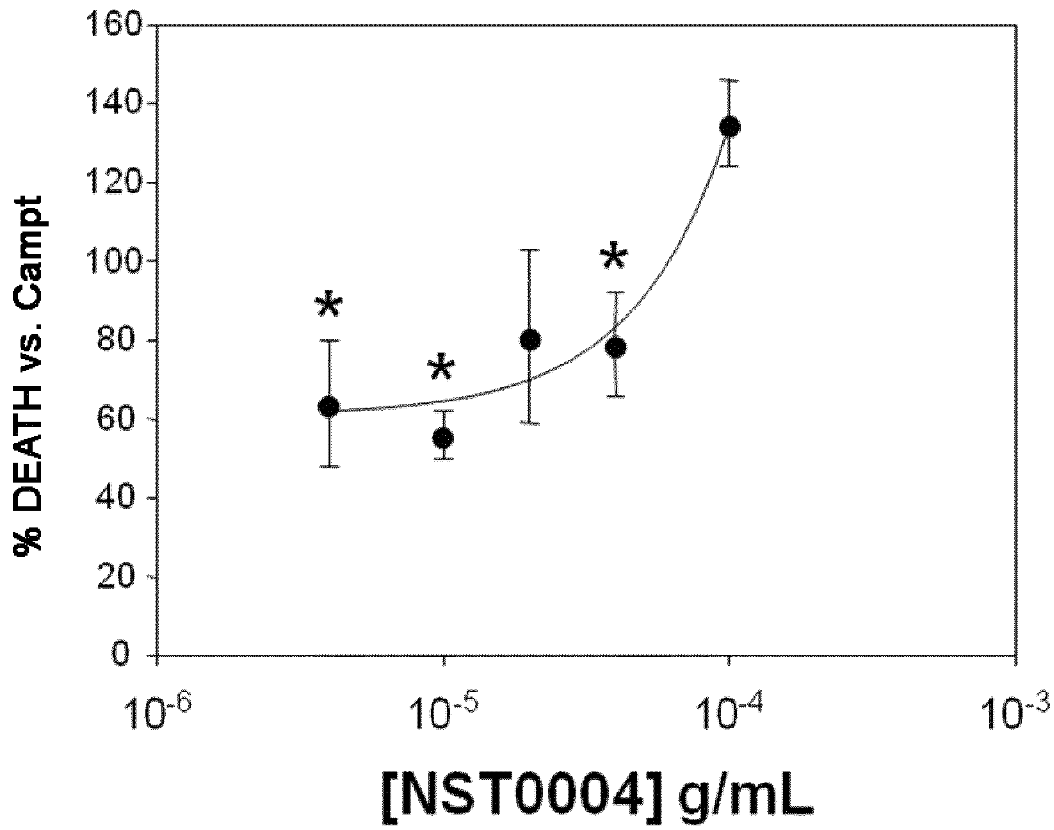
160
140
120
100
80
60
40
20
0
% DEATH vs. Campt
*
*
*
10-6
10-5
10-4
10-3
[NST0004] g/mL

Figure 5

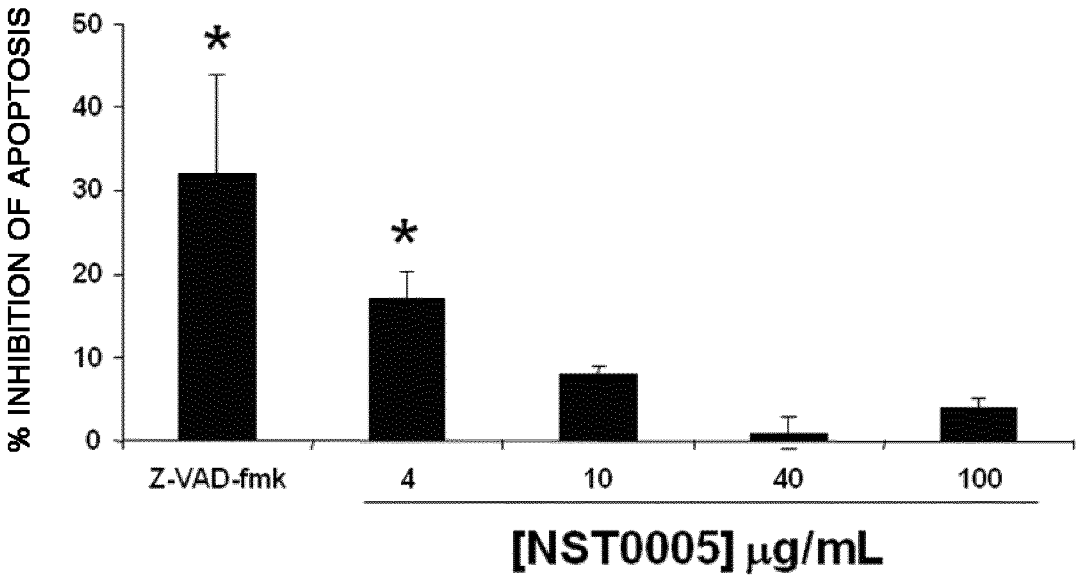
% INHIBITION OF APOPTOSIS
50
40
30
20
10
0
*
*
Z-VAD-fmk
4
10
40
100
[NST0005] µg/mL

Figure 6

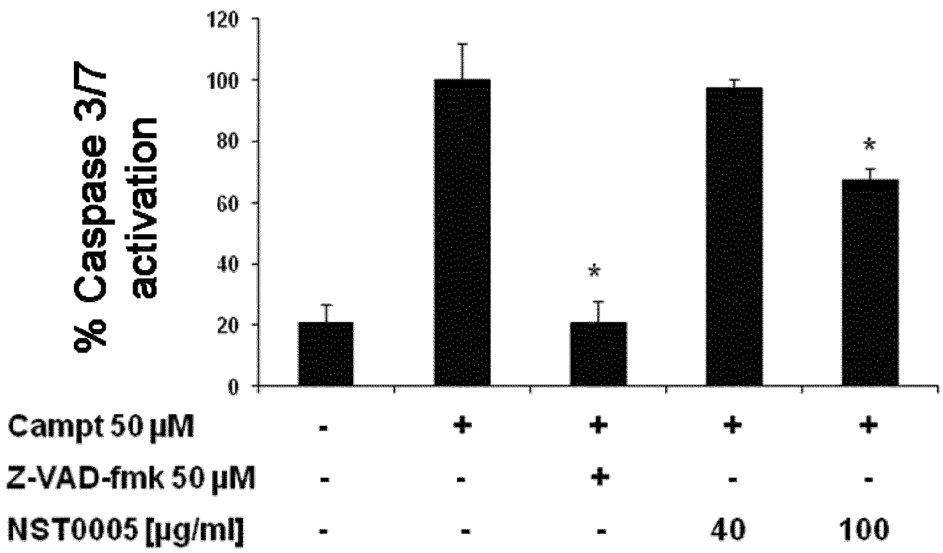
% Caspase 3/7 activation
120
100
80
60
40
20
0
*
*
Campt 50 µM - + + + +
Z-VAD-fmk 50 µM - - + - -
NST0005 [µg/ml] - - - 40 100

Figure 7

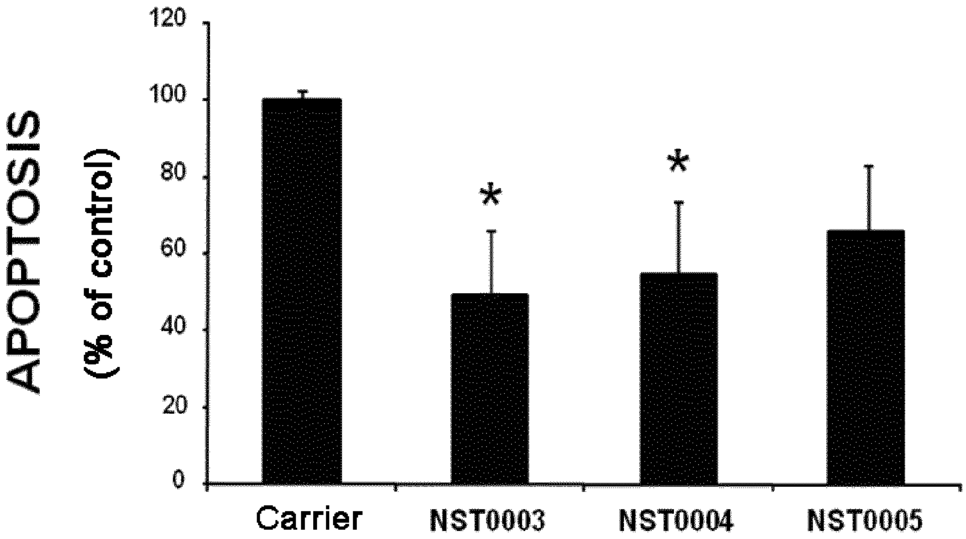


% Apoptosis vs. TBH

Figure 8

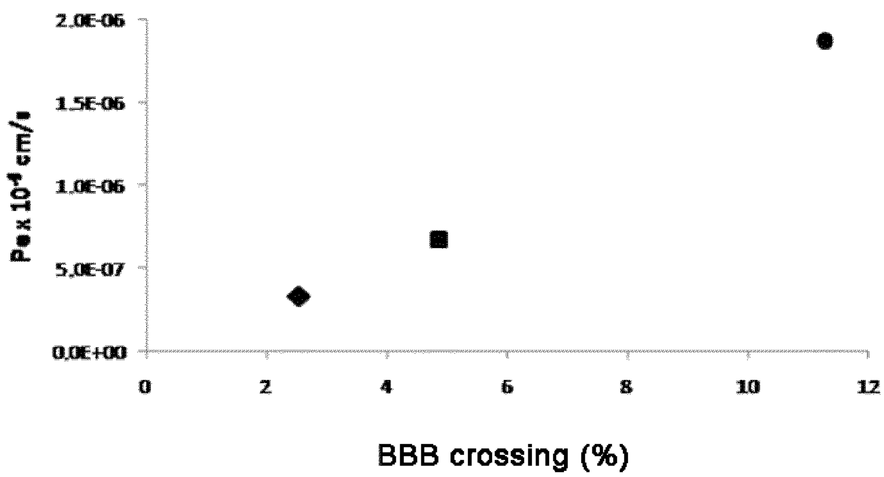


| | BBB crossing (%) | $P_e$ (cm/s) | n | cLogP |
|---|---|---|---|---|
| ◆ NST0003 | 2.6±2.1 | $0.3 \pm 0.2 \times 10^{-6}$ | 12 | 3.29 |
| ■ NST0004 | 4.9±1.9 | $0.7 \pm 0.2 \times 10^{-6}$ | 15 | 3.63 |
| ● NST0005 | 11.3±2.8 | $1.9 \pm 0.3 \times 10^{-6}$ | 9 | 4.14 |

Figure 9

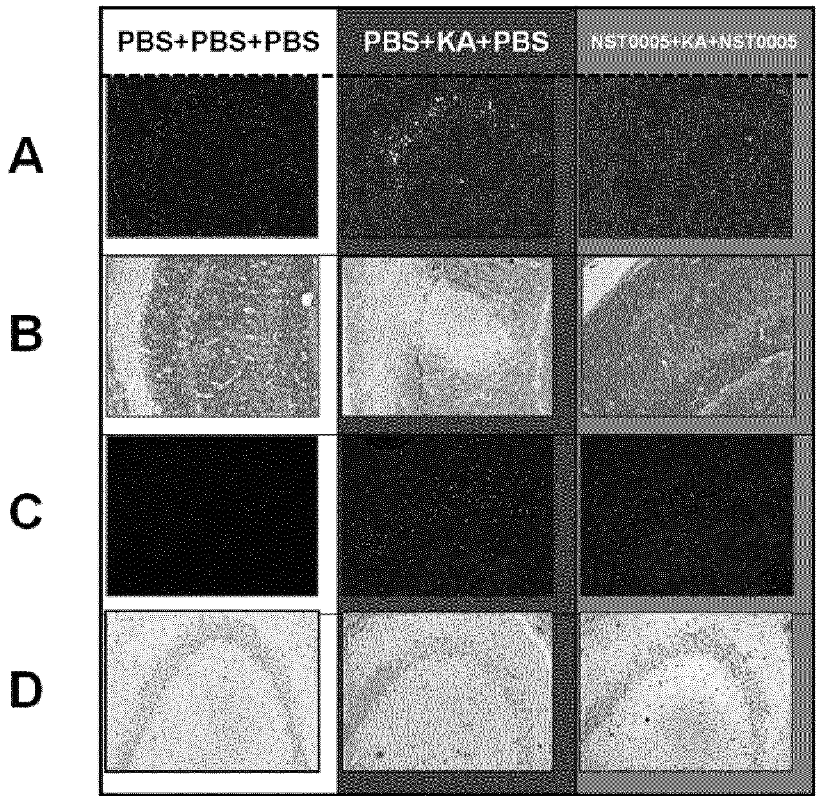


Figure 10

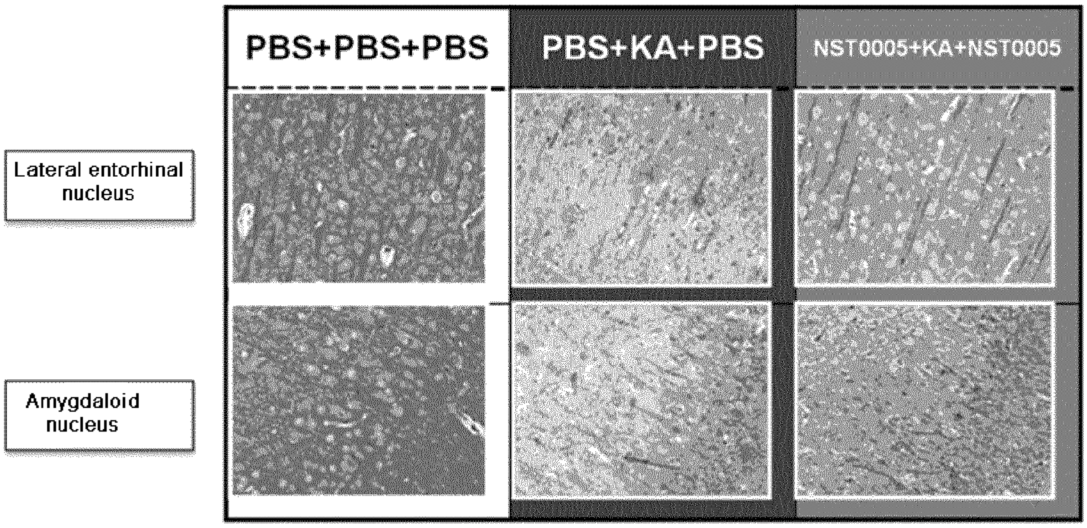


Figure 11

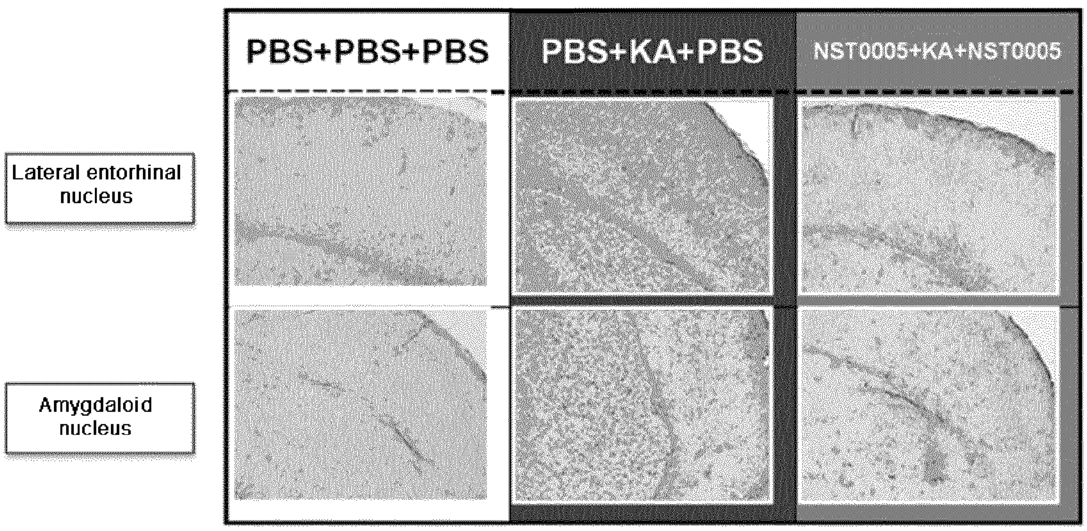


Figure 12

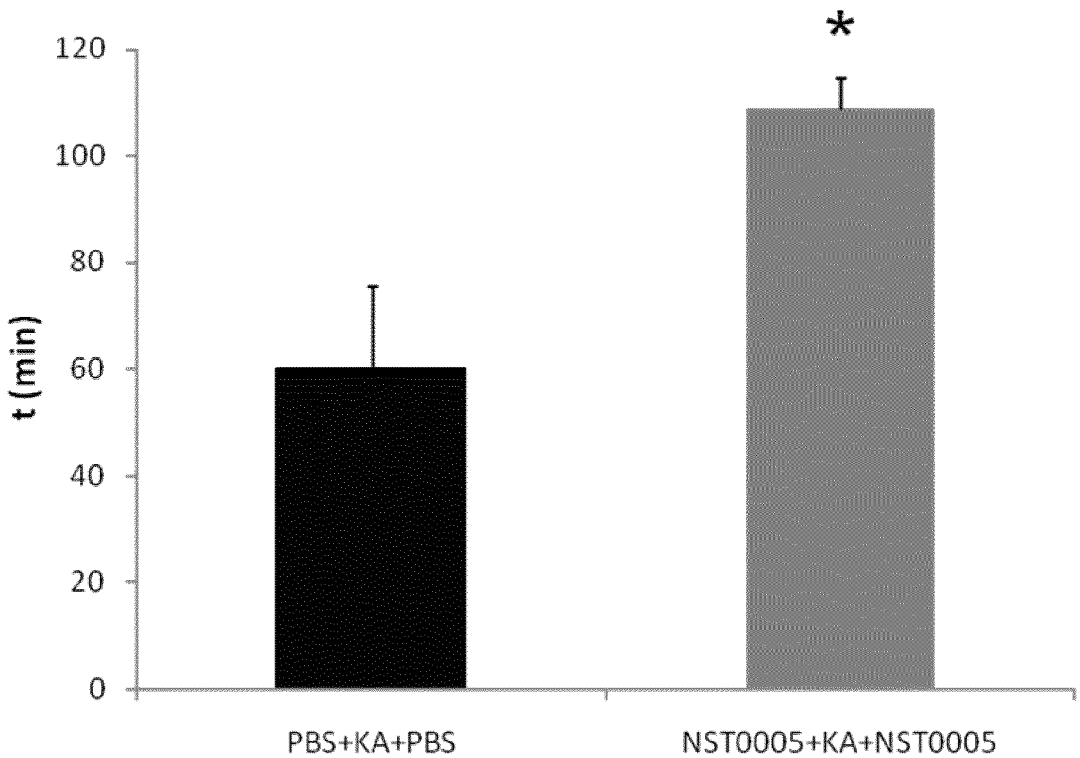


Figure 13

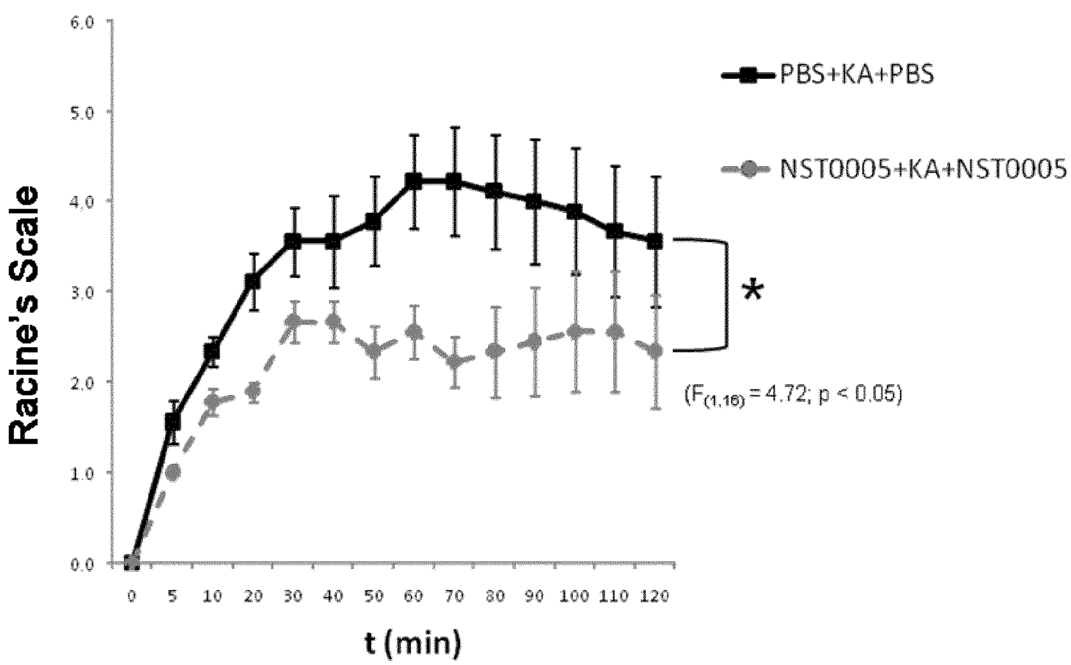


Figure 14

## INTERNATIONAL SEARCH REPORT

International application No.
PCT/ ES 2010/070126

A. CLASSIFICATION OF SUBJECT MATTER

see extra sheet

According to International Patent Classification (IPC) or to both national classification and IPC

B. FIELDS SEARCHED

Minimum documentation searched (classification system followed by classification symbols)

Documentation searched other than minimum documentation to the extent that such documents are included in the fields searched

Electronic data base consulted during the international search (name of data base and, where practicable, search terms used)

INVENES,EPODOC, WPI, REGISTRY, HCAPLUS, MEDLINE, EMBASE, BIOSIS

C. DOCUMENTS CONSIDERED TO BE RELEVANT

| Category* | Citation of document, with indication, where appropriate, of the relevant passages | Relevant to claim No. |
|---|---|---|
| X | Base of datos Registry [retrieved on 27.04.2010]. STN International, Columbus, Ohio (EEUU). Compuesto con CAS Registry Number RN 160522-03-2. Fecha of intrada in Registry 31-01-1995. | 1,2,5 |
| | Compuesto con CAS Registry Number RN 79902-42-4. Fecha of intrada in Registry 16-11-1984. | 1,3,5 |
| | Compuesto con CAS Registry Number RN 79902-39-9. Fecha of intrada in Registry 16-11-1984. | 1,4,5 |

☒ Further documents are listed in the continuation of Box C. ☒ See patent family annex.

* Special categories of cited documents:

"A" document defining the general state of the art which is not considered to be of particular relevance.

"E" earlier document but published on or after the international filing date

"L" document which may throw doubts on priority claim(s) or which is cited to establish the publication date of another citation or other special reason (as specified)

"O" document referring to an oral disclosure use, exhibition, or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other documents , such combination being obvious to a person skilled in the art

"&" document member of the same patent family

| Date of the actual completion of the international search | Date of mailing of the international search report |
|---|---|
| 29 April 2010 (29.04.2010) | (20/05/2010) |
| Name and mailing address of the ISA/ O.E.P.M.<br>Paseo de la Castellana, 75 28071 Madrid, España.<br>Facsimile No. 34 91 3495304 | Authorized officer<br>E. Albarrán Gómez<br>Telephone No. +34 91 349 30 38 |

Form PCT/ISA/210 (second sheet) (July 2009)

## INTERNATIONAL SEARCH REPORT

| International application No. |
|---|
| PCT/ES 2010/070126 |

C (continuation). DOCUMENTS CONSIDERED TO BE RELEVANT

| Category* | Citation of documents, with indication, where appropriate, of the relevant passages | Relevant to claim No. |
|---|---|---|
| A | LEE, J.-K. and col. Statin inhibits kainic acid-induced seizure and associated inflammation and hippocampal cell death. Neuroscience Letters. August 2008, Vol. 440, Nº 3, pages 260-264. the whole document, in especial, page 260; page 261, figure 1B; page 262, figure 2C and 2D. | 1-6 |
| A | VOLLMER, T. and col. Oral Simvastatin treatment in relapsing-remitting multiple sclerosis. The Lancet. 15 May 2004, Vol. 363, pages 1607-1608. the whole document. | 1-6 |
| A | CORDLE, A. & LANDRETH, G. 3-Hydroxy-3-Methylglutaryl-Coenzyme A Reductase Inhibitors Attenuate Beta-Amyloid-Induced Microglial Inflammatory Responses. The Journal of Neuroscience. 2005, Vol. 25, Nº 2, pages 299-307. the whole document. | 1-6 |
| A | WOLOZIN, B. and col. Simvastatin is associated with a reduced incidence of dementia and Parkinson´s disease. BMC Medicine. 2007, Vol. 5, Nº 20, pages 1-11. the whole document. | 1-6 |
| P,A | WO 2009147275 A1 (NEURON BIOPHARMA, S.A.) 10.12.2009, the whole document. | 1-6 |

Form PCT/ISA/210 (continuation of second sheet) (July 2009)

## INTERNATIONAL SEARCH REPORT

Information on patent family members

International application No.

PCT/ ES 2010/070126

| Patent document cited in the search report | Publication date | Patent family member(s) | Publication date |
|---|---|---|---|
| WO 2009147275 A | 10.12.2009 | ES 2330184 A | 04.12.2009 |

Form PCT/ISA/210 (patent family annex) (July 2009)

**INTERNATIONAL SEARCH REPORT**

International application No.

PCT/ ES 2010/070126

CLASSIFICATION OF SUBJECT MATTER

***A61K 31/35*** (2006.01)
***A61P 25/28*** (2006.01)
***A61P 25/08*** (2006.01)

Form PCT/ISA/210 (extra sheeet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4866090 A


### Non-patent literature cited in the description


- **Blennow et al.** *Lancet,* 2006, vol. 368, 387-403
- **Pahan.** *Cell Mol Life Sci.,* 2006, vol. 63 (10), 1165-78
- **Ling et al.** *Ann Neurol.,* 2006, vol. 60 (6), 729-39
- **Lee et al.** *Neurosci Lett.,* 2008, vol. 440, 260-4
- **Fandino et al.** *Neurocirugia.,* 2007, vol. 18, 16-27
- **Honjo et al.** *Arch. Ophthalmol.,* 2002, vol. 120, 1707-13
- **Racine.** *Electroencephalogr Clin Neurophysiol,* 1972, vol. 32 (3), 281-94
- **C. Fauli ; Trillo.** Tratado de Farmacia Galénica. 2003
- **Hoffman et al.** *J. Med. Chem.,* 1986, vol. 29, 849-852
- **Westerfield, M.** The Zebrafish Book. A Guide for the Laboratory Use of Zebrafish (Danio rerio). University of Oregon Press, 1995
- **Nusslein-Volhard, C ; Dahm, R.** Zebrafish. Practical approach. Oxford University Press, 2002
- Guidance on the Operation of Animals. *Scientific Procedures, Act.,* 1986